# EUROPÄISCHE PATENTSCHRIFT

(11) **EP 2 981 301 B1**
(45) Veröffentlichungstag und Bekanntmachung des Hinweises auf die Patenterteilung: **30.11.2016**
(21) Anmeldenummer: 14795823.5
(22) Anmeldetag: 07.11.2014
(51) Int. Cl.: A61L 15/18, A61F 13/00, A61L 15/42, A61L 15/44

(54) **MEDIZINISCHE AUFLAGE**
MEDICAL DRESSING
EMPLÂTRE MÉDICAL

(30) Priorität: 07.11.2013 DE 102013018642
(43) Veröffentlichungstag der Anmeldung: 10.02.2016
(73) Patentinhaber: BSN medical GmbH, 20253 Hamburg (DE)
(72) Erfinder: SUSCHEK, Christoph V., 40764 Langenfeld (DE)
(74) Vertreter: Jostarndt Patentanwalts-AG
(86) Internationale Anmeldenummer: PCT/EP2014/074029
(87) Internationale Veröffentlichungsnummer: WO 2015/067746

(56) Entgegenhaltungen:
- WO-A2-2007/084533
- CN-A- 103 127 504
- US-A1- 2009 108 777

## Beschreibung

Die vorliegende Erfindung betrifft eine NO-freisetzende medizinische Auflage. Diese Auflage ist insbesondere aus mindestens zwei Lagen modular aufgebaut und ist in der Lage, mittels der Emission elektromagnetischer Strahlung aus einem Lichtmodul, in einem benachbarten, bevorzugt enganliegenden, Absorptionsmodul eingelagerte photolabile Stickstoffmonoxid-Vorstufen zu spalten, so dass das photolytisch erzeugte Stickstoffmonoxid zur Unterstützung medizinischer Therapien bei Tier und Mensch sowie zur Generierung von NO verwendet werden kann.

Die Behandlung von Durchblutungsstörungen und den daraus resultierenden chronischen Wunden ist im klinischen Alltag nach wie vor unzulänglich. Diese Beschwerden sind nicht nur ein ernstes medizinisches, sondern auch ökonomisches Problem. So wird geschätzt, dass alleine in der BRD etwa 2,4 Millionen Diabetiker unter einer Durchblutungsstörung und/oder einer gestörten Wundheilung leiden. Die Lebensqualität der Kranken ist eingeschränkt, und sie leiden unter vermeidbaren Schmerzen. Die Behandlungskosten werden auf 3 Milliarden Euro jährlich geschätzt. Und mit der Alterung der Bevölkerung werden künftig noch weit mehr Menschen an solchen schwer heilenden Wunden leiden. So gehen Schätzungen von einer Verdopplung der Zahlen bis 2025 aus.

Die aktuelle Therapie stützt sich vor allem auf die mäßig effiziente pharmakologische Unterstützung der Gewebedurchblutung, sowie auf eine für die Heilung chronischer Wunden unzureichende Unterstützung durch Wundauflagensysteme.

Ein wichtiges physiologisches Prinzip der menschlichen Haut ist die enzymatische Produktion von Stickstoffmonoxid mit Hilfe von Enzymen aus der Familie der NO-Synthasen, die von allen Zelltypen synthetisiert werden können [1]. Das Substrat der NO-Synthasen ist die Aminosäure L-Arginin. Man unterscheidet heute zwischen zwei konstitutiv exprimierten und einer induzierbaren Isoform der NO-Synthasen. Zu den konstitutiv exprimierten NO-Synthasen zählen die überwiegend neuronal lokalisierte NO-Synthase (nNOS) und die vorwiegend endothelial lokalisierte NO-Synthase (eNOS), die jedoch auch in dermalen Fibroblasten und im Hautmuskelschlauch exprimiert wird, wohingegen die induzierbare Isoform, die iNOS, erst durch die Wirkung von proinflammatorischen Stimuli induziert wird und im Gegensatz zu den konstitutiven Isoformen über einen längeren Zeitraum (Tage) lokal hohe Konzentrationen von NO produzieren kann.

Im Folgenden werden die Begriffe Stickstoffmonoxid, Stickstoffoxid, Stickstoffmonoxidradikal, NO und NO• als gleichbedeutende Begriffe für das gleiche Molekül benutzt.

Daneben kann das NO auch nicht-enzymatisch aus Nitrit oder Nitrosothiolen freigesetzt werden. Die nicht-enzymatische NO-Generierung findet unter sauren und reduzierenden Bedingungen statt. Dabei wird z. B. aus Nitrit NO freigesetzt. Physiologisch bedeutsam ist diese Reaktion im sauren Milieu des Magens sowie der Haut. Es ist zudem bekannt, dass UVA-Licht aus Nitrit eine Substanz mit den physiologischen Eigenschaften von NO freisetzen kann [2]. Tatsächlich konnte demonstriert werden, dass NO über den Weg des lichtenergieinduzierten Zerfalls aus Nitrit entstehen kann [3; 4].

NO steuert im Rahmen entzündlicher Prozesse der Haut, im Zusammenspiel der unterschiedlichen Zellsysteme u. a. die Proliferation, die Differenzierung von Hautzellen und somit z. B. auch die Wundheilung [5]. In Wundheilungsprozessen der Haut sind eine Reihe von Genen dominant als NO-reguliert identifiziert worden [6; 7] und entsprechend stellte sich die Wundheilung in iNOS-defizienten Mäusen als ein signifikant verzögerter Prozess dar [8]. Weitere Gene, die unter der Transkriptionskontrolle durch NO stehen, sind protektiv wirkende Stress-Schutz-Gene wie Heat-Shock-Proteine, Chaperone oder auch die Hämoxygenase-1. Ein weiterer Teil von NO-regulierten Genen dient entweder der Gegenregulation der Entzündungsreaktion oder der Reparatur von lokalen Schäden (hierzu zählen insbesondere viele Mitglieder der Familie der Matrix-Metalloproteinasen (MMP)). NO kann die Genexpression der MMPs, aber auch ihrer physiologischer Inhibitoren, der tissue inhibitors of matrix proteinases (TIMP), beeinflussen und überdies durch Nitrosierung deren Aktivität modulieren und somit einem vermehrten Kollagenabbau durch die MMPs entgegenwirken [9]. Daneben beeinflusst NO auch die Expression und Aktivität von Wachstumsfaktoren, wie z. B. dem VEGF [37; 38]. So konnte z. B. die Angiogenese, neben der Kollagensynthese eine Schlüsselkomponente der Wundheilung, durch NO-Donoren angeregt werden [39], wobei NO in Keratinozyten und Makrophagen die Synthese des Angiogenesefaktors VEGF zu induzieren vermag [5; 40].

Zudem wurde in Versuchen mit exogenen NO-Donoren gezeigt, dass NO zu einer signifikanten Erhöhung der Kollagensynthese in Fibroblasten führt [10; 11]. Ein wichtiger physiologischer Induktor der Kollagenneusynthese ist der transforming growth factor-β (TGF-β), wohingegen Interleukin-1 (IL-1), IL-6, TNF-α sowie reaktive Sauerstoffspezies (ROS) die Kollagenneusynthese signifikant mindern oder sogar hemmen können [12; 13]. NO kann auch aufgrund seiner Fähigkeit, mit anderen Radikalen zu reagieren und diese so zu eliminieren, protektiv wirken [14]. So ist NO in der Lage vor hydroxylradikalinduzierten DNA-Schäden und vor H₂O₂-induziertem Zelltod zu schützen und es besitzt eine größere Kapazität zur Terminierung der radikalinduzierten Lipidperoxidation als Vitamin E [15; 16].

Daneben werden auch zahlreiche andere protektive Eigenschaften von NO beschrieben. So soll NO vor hypoxieinduzierten Schäden schützen, es entfaltet hepatozyten- und neuroprotektive Wirkungen und über eine Inaktivierung von Effektorcaspasen kann es vor Apoptose schützen [17]. Zudem kann NO bereits in geringen Konzentrationen wichtige Komponenten des antioxidativen Schutzes wie z. B. den Glutathionstoffwechsel (GSH) modulieren, indem es eine Erhöhung der Expression der beiden Schlüsselenzyme der GSH-Synthese, der γ-Glutamyl-Cystein-Synthetase (γ-GCS) und der γ-Glutamyl-Transpeptidase induziert [18].

Einmal gebildet, diffundiert NO leicht sowohl in die Gefäßwand als auch in das Gefäßlumen und ist z. B. beteiligt an der Regulation der Thrombozytenadhäsion und Thrombozytenaggregation, des vaskulären Rollings sowie der Transmigration von neutrophilen Granulozyten und Monozyten, als auch der endothelialen Permeabilität [20]. NO relaxiert zudem in der Gefäßwand die glatten Muskelzellen über eine Aktivierung der löslichen Guanylatzyklase, dem Schlüsselenzym in der Regulation des Blutdrucks. Das endothelial gebildete NO hat somit eine essentielle Bedeutung für die Aufrechterhaltung sowohl der Gefäßfunktion als auch der Gefäßstruktur und beeinflusst somit wesentlich hämodynamische Parameter, insbesondere den Blutdruck, aber auch gewebsischämische Zustände [21; 22].

Bei einer verminderten NO-Syntheserate wird im Tiermodell eine verzögerte Gefäßneubildung und Wundheilung sowie eine stark gestörte Reepithelisierung von Hautwunden aufgrund einer verminderten Proliferationsrate der Keratinozyten beobachtet. Als Transmitter der Gefäßrelaxation kann NO die Blutflussrate im Wundareal erhöhen und somit zu einer vermehrten Sauerstoff- und Nährstoffversorgung sowie einer gesteigerten zellulären Infiltration des Gewebes führen [5].

Die topische Behandlung von Wunden mit NO-Donoren, während der frühen Phase der kutanen Wundheilung führt zu einer signifikant beschleunigten Wundschließung und Reepithelisierung in der Ratte [23], wie auch zu einer verbesserten Wundheilung bei Mäusen mit diabetischen Hintergrund [24]. Auch die tägliche topische Exposition von Wunden mit NO-haltigem Luft-Plasma verbesserte signifikant die Wundheilung septischer sowie aseptischer Wunden im Rattenmodell [25]. Trotz der zahlreichen Hinweise auf den positiven Einfluss von NO auf die Wundheilung wurde am Menschen bis dato nur eine Pilotstudie mit einem 55-Jährigen Patienten dokumentiert, bei dem eine NO-Gas Therapie zur vollständigen Heilung eines mehrere Jahre alten, therapieresistenten Ulcus cruris venosum am Fuß führte [26]. Exogen verabreichtes NO kann durch den Abbau von reaktiven Sauerstoffspezies Ischämie/Reperfusion-bedingte Schäden mildern und die Mikrozirkulation des Hautgewebes wesentlich verbessern. Diese Eigenschaften spielen eine besondere Rolle in der Revitalisierung von Randgebieten von freien Lappenplastiken im Rahmen von Weichteilgewebsdeckungen [26]

Aktuelle, den NO-Haushalt-betreffende Therapieansätze versuchen überwiegend die durch NO induzierte cGMP-abhängige Signalkaskade zu adressieren. Therapieansätze zu direkten Beeinflussung der NO-Verfügbarkeit im Organismus begrenzen sich auf die Verwendung organischer Nitrite und Nitrate [27]. NO-Gas wird in der Klinik bis dato nur inhalativ in der Therapie verschiedener akuter Lungendysfunktionen verwendet, wobei in experimentellen Studien auch eine systemische Wirkung inhalierten NOs nachgewiesen werden konnte [28]. Der Diffusionskoeffizient von NO bei 37 °C ist ca. 1,4-fach höher als der von Sauerstoff oder Kohlenmonoxid, wonach eine für das NO-Molekül erreichbare Diffusionsstrecke in Geweben von 500 µm errechnet wurde [29].

Ghaffari et al. konnte signifikante antibakterielle Wirkungen und somit die Relevanz in der Behandlung von mit Bakterien infizierten Wunden und Brandverletzungen sowie nicht heilenden Wunden durch exogenes NO-Gas nachweisen [31; 32], wobei die verwendeten NO-Konzentrationen in vitro keine toxische Effekte auf humane Fibroblasten, Keratinozyten oder Endothelzellen zeigten [33].

Zusammenfassend hat sich hiermit das Stickstoffmonoxid (NO) als physiologisch wichtiges bioaktives Molekül herausgestellt. Durch seine erweiternde Wirkung auf die Blutgefäße, die zudem sehr schnell auftritt, besitzt das NO eine große Bedeutung für die Blutversorgung von Organen. Darüber hinaus spielt das NO auch in anderen physiologischen Prozessen eine Rolle als wichtiger Botenstoff. So schützt das NO als Radikalfänger vor Hypoxie-induzierten Schäden und moduliert wichtige Komponenten des antioxidativen Schutzes. Beachtlicherweise steuert das NO bei entzündlichen Prozessen der Haut im Zusammenspiel der unterschiedlichen Zellsysteme beispielsweise die Proliferation und Differenzierung von Hautzellen und fördert so die Wundheilung.

In entsprechender Weise zeigt sich im Tiermodell, dass eine verminderte NO-Syntheserate mit einer verzögerten Gefäßneubildung und Wundheilung einhergeht.

Basierend auf den Erkenntnissen zu NO gibt es bereits Ansätze, gasförmiges NO für die Therapie von Durchblutungsstörungen oder chronischen Wunden einzusetzen. Bisher wird ein für therapeutische Zwecke genutztes NO-haltiges Gas aus Gasflaschen (Industriegas) bereitgestellt, deren Lagerung und Handhabung in einer Klinik oder einer anderen Therapieeinrichtung aufgrund der erforderlichen Sicherheitsmaßnahmen aufwendig ist. Dies gilt insbesondere für eine mobile Vorrichtung. Zudem muss die Qualität des gelagerten Gases für medizinische Anwendungen hohen Anforderungen genügen, die den Aufwand für Herstellung und Lagerung weiter erhöhen. Schon eine geringe Verunreinigung des Gases führt zur Bildung unerwünschter und eventuell giftiger Nebenprodukte. Die europäischen Arzneimittel- und Gesundheitsbehörden haben demgemäß hohe Anforderungen an die Reinheit des zu verwendenden Stickstoffmonoxids gestellt. Neben der Verwendung von "technischen" NO-Gasen für die medizinische Anwendung, gibt es Verfahren zur plasmachemischen Erzeugung von Stickstoffmonoxid. Diese Verfahren erfordern nachgeschaltete, teilweise sehr aufwändige, Reinigungsverfahren, und es ist schwierig, eine optimale Konzentration von NO für den jeweiligen therapeutischen Zweck einzustellen.

US 2009/108777 A1 offenbart ein Medizinische Auflage umfassend ein Strahlung-emittierendes Modul (SEM) mit einer Strahlungsquelle zur Emission elektromagnetischer Strahlung, und ein NO-Modul (NOM), das photolabile Stickstoffmonoxidvorstufen (NOD) enthält, wobei die aus dem SEM emittierte elektromagnetische Strahlung diese NOD spalten kann, so dass im NOM NO generiert wird, welches aus dem NOM freigesetzt werden kann.

Es besteht daher noch Bedarf an neuen Verfahren zur Behandlung von Durchblutungsstörungen und chronischen Wunden.

Aufgabe der Erfindung ist es daher, einen neuen therapeutischen Ansatz zur Behandlung von Durchblutungsstörungen und chronischen Wunden bereitzustellen, der bezüglich mindestens einer der oben genannten Nachteile verbessert ist.

### Zusammenfassung der Erfindung

Diese Aufgabe wird gemäß der Erfindung dadurch gelöst, dass eine medizinische Auflage bereitgestellt wird, die folgendes umfassend:
a. ein Strahlung-emittierendes Modul (SEM) mit einer Strahlungsquelle zur Emission elektromagnetischer Strahlung, und
b. ein NO-Modul (NOM), das photolabile Stickstoffmonoxidvorstufen (NOD) enthält, wobei die aus dem SEM emittierte elektromagnetische Strahlung diese NOD spalten kann, so dass im NOM NO generiert wird, welches aus dem NOM freigesetzt werden kann;
   wobei in der Auflage ein System eingesetzt wird, welches mehrfach oxidierte Stickoxide, Sauerstoffradikalanionen, Hydroxylradikale oder aquatisierte Elektronen abbaut oder neutralisiert.

Erfindungsgemäß umfasst die medizinische Auflage somit:
a. ein Strahlung-emittierendes Modul (SEM) mit einer Strahlungsquelle zur Emission elektromagnetischer Strahlung, und
b. ein NO-Modul (NOM), das photolabile Stickstoffmonoxidvorstufen (NOD) enthält, wobei die aus dem SEM emittierte elektromagnetische Strahlung diese NOD spalten kann, so dass im NOM NO generiert wird, welches aus dem NOM freigesetzt werden kann;
c. ein System, welches mehrfach oxidierte Stickoxide, Sauerstoffradikalanionen, Hydroxylradikale oder aquatisierte Elektronen abbaut oder neutralisiert.

Die erfindungsgemäße medizinische Auflage vereinigt mehrere entscheidende Vorteile gegenüber den aus dem Stand der Technik bekannten therapeutischen Ansätzen.

Es zeigte sich in überraschender Weise, dass auch im Rahmen einer solchen medizinischen Auflage durch eine System, welches mehrfach oxidierte Stickoxide, Sauerstoffradikalanionen, Hydroxylradikale oder aquatisierte Elektronen abbaut oder neutralisiert (im Rahmen der Erfindung auch als "Radikalfänger-System" bezeichnet), auf zuverlässige Weise NO hergestellt werden kann, das frei von Verunreinigungen ist und entsprechend für eine medizinische Anwendung geeignet ist.

Die medizinische Auflage erlaubt eine transdermale Applikation von NO, die mit mehreren Vorteilen einhergeht, so werden insbesondere eine gastrointestinale Unverträglichkeit und ein hepatischer First-Pass-Effekt umgangen.

Darüber hinaus kann durch die NO induzierte Vasodilatation der Haut-Mikrozirkulation die perkutane Aufnahme von pharmakologisch aktiven Substanzen signifikant erhöht werden. Das NO wirkt in dieser Hinsicht als Penetrationsverstärker oder Transportvermittler.

Aufgrund des limitierten Lösungsverhaltens von NO können in dem NOM als Absorptionsmodul zwar physiologisch relevante NO Konzentrationen generiert werden, die jedoch weit unter denen liegen, die für den Menschen gesundheitliche Schäden verursachen könnten.

Ferner kann durch einen direkten Kontakt der menschlichen Körperoberfläche mit dem photolytisch erzeugten Stickstoffmonoxid-freisetzenden Modul der Vorrichtung eine wesentlich genauere NO-Behandlung erreicht werden als z. B. mit NO-haltigen Gasgemischen bzw. spontan zerfallenden NO-Donoren.

Üblicherweise erschwert die kurze Halbwertszeit des NO den therapeutischen Einsatz. Mit der erfindungsgemäßen Vorrichtung kann trotz der kurzen Halbwertszeit durch eine kontinuierliche NO-Nachsynthese aus den NOD ein gleichbleibender NO-Spiegel aufrechterhalten werden.

Diese Fähigkeit zur Regulierung und Steuerung ist gerade im therapeutischen Bereich von entscheidendem Vorteil, da sie eine auf den jeweiligen Patienten abgestimmte Behandlung ermöglicht.

Der modulare Aufbau der medizinischen Auflage ermöglicht auch den Einsatz eines NOD-haltigen Moduls als austauschbarer Verbrauchsartikel, der eine reproduzierbare und sichere Herstellung von NO gewährleisten kann.

Durch einfache Anpassung der NOM bezüglich Größe, Form und Material kann die medizinischen Auflage gezielt an die Behandlungserfordernisse angepasst werden.

So kann durch die Verwendung von NO-undurchlässigen Schichten, hier insbesondere einer Rückschicht oder von randseitigen Klebeschichten, das NO gezielt dem zu exponierenden Hautareal zugeführt werden und gelangt somit nicht in die Umwelt.

Da das NO-generierende Modul einen Bestandteil der erfindungsgemäßen medizinischen Auflage darstellt, kann auf eine externe Zuführung von NO, wie es meist durch Gasflaschen geschieht, verzichtet werden.

Dies erlaubt den Einsatz als mobiles System, das gerade im therapeutischen Bereich eine Anwendung außerhalb von Praxen oder Kliniken ermöglicht und damit insbesondere bei chronischen Krankheiten mit einer kostengünstigeren Behandlung und einer höheren Patienten-Compliance einhergeht.

Bei der erfindungsgemäßen Auflage handelt es sich um eine einfach aufgebaute medizinische Auflage mit handelsüblichen Komponenten, so dass es nicht nur kostengünstig in der Herstellung ist, sondern auch bei geringer Fehleranfälligkeit einfach in der Anwendung ist.

Zusammenfassend stellt die erfindungsgemäße Auflage eine NO-basierte Therapieform dar, bei der preiswert, zuverlässig, sicher und für den Patienten individualisierbar NO von einer medizinischen Auflage freigesetzt wird.

### Die Erfindung im Einzelnen

In einem zweiten Aspekt stellt die Erfindung eine medizinische Auflage bereit, die ein NO-Modul (NOM) umfasst, das photolabile Stickstoffmonoxidvorstufen (NOD) enthält, wobei elektromagnetische Strahlung diese NOD spalten kann, so dass im NOM NO generiert wird, welches aus dem NOM freigesetzt werden kann. Hierbei enthält das NOM ein System, das mehrfach oxidierte Stickoxide, Sauerstoffradikalanionen oder Hydroxylradikale abbaut oder neutralisiert (Radikalfänger-System).

In einem dritten Aspekt stellt die Erfindung eine medizinische Auflage bereit, die ein NO-Modul (NOM) umfasst, das photolabile Stickstoffmonoxidvorstufen (NOD) enthält, wobei das NOM zusätzlich Übergangsmetallkationen enthält, die durch Reduktion aus den NOD das NO freisetzen können (sog. NO-aktive Übergangsmetallkationen).

Im Rahmen der vorliegenden Erfindung sind unter den Übergangsmetallen die chemischen Elemente mit den Ordnungszahlen von 21 bis 30, 39 bis 48, 57 bis 80 und 89 bis 112 zu verstehen.

Es hat sich gezeigt, dass in der medizinischen Auflage bei der Strahlungsinduzierten NO-Generierung durch die anwesenden NO-aktiven Übergangsmetallkationen, NO in weitaus höherer Ausbeute generiert werden kann und die Bildung von reaktiven Sauerstoffspezies sowie höher oxidierten Stickoxidspezies nicht stattfindet, so dass das erhaltene NO von hoher Reinheit ist. In diesem Aspekt der Erfindung kann somit in der medizinischen Auflage auf das Radikalfänger-System verzichtet werden.

Weiterhin hat sich gezeigt, dass diese Übergangsmetallkationen auch schon ohne elektromagnetische Strahlung durch alleinige Reduktion der NO-Vorstufe das NO freisetzen können. Damit ist einerseits möglich, dass bei der Anwendung auf die Strahlungsquelle verzichtet werden kann, andererseits bedingt dies aber, dass die beiden Reaktionskomponenten, also das NO-aktive Übergangsmetallkation und die NOD erst bei der Anwendung zusammentreffen dürfen. Hierzu sind verschiedene Ausführungsformen möglich:
Das NO-aktive Übergangsmetallkation wird aus einer inaktiven Wertigkeitsstufe des entsprechenden Kations durch Reduktion oder Oxidation in situ hergestellt. Hierbei muss gewährleistet sein, dass die Redoxreaktion erst bei der Anwendung abläuft. Dies kann durch Trennung des Redoxmittels von dem inaktiven Kation gewährleistet werden, wobei bei der Anwendung durch Vermischen der Komponenten die Reaktion gestartet wird. Alternativ können auch das NO-aktive Übergangsmetallkation und die NOD in dem NOM voneinander getrennt vorliegen.

In einer bevorzugten Ausführungsform wird das NO-aktive Übergangsmetallkation durch eine Licht-induzierte Redoxreaktion im NOM hergestellt.

In einer Ausführungsform handelt es sich bei dem NO-aktiven Übergangsmetallkation um ein Kation von niedriger Wertigkeit, d.h. dass es für das entsprechende Kation auch ein Kation mit einer höheren Wertigkeit gibt.

Diese beiden Wertigkeiten des Übergangsmetallkations bilden hierbei ein Redoxpaar, wobei das Kation niedriger Wertigkeit bei der Reduktion der NO-Vorstufe in das Kation mit der höheren Wertigkeit umgewandelt wird. Diese beiden Kationen bilden somit das Redoxpaar einer NO-generierenden Reaktion, die sich wie folgt formulieren lässt:

Meⁿ⁺ + NO₂⁻ + 2H⁺ → Me⁽ⁿ⁺¹⁾⁺ + NO^{•} + H₂O

Bevorzugt werden hierbei als Kationen Fe²⁺, Co⁺, Ru²⁺ oder Cu⁺ eingesetzt. Diese können in Form von anorganischen oder organischen Salzen in dem NOM vorhanden sein.

In bevorzugter Weise werden für die Reaktion mit den Übergangsmetallkationen als NOD Nitrite oder S-Nitrosothiole verwendet. Eine entsprechende Reaktion von Cu+ mit Nitrit findet wie folgt statt:

Cu⁺ + NO₂⁻ + 2H⁺ → Cu²⁺ + NO^{•} + H₂O

Da bei dieser Redoxreaktion das Kation niedriger Wertigkeit entsprechend der damit einhergehenden NO-Generierung verbraucht wird, ist es zweckmäßig, entweder dieses Kation in einem Überschuss vorzuhalten oder es durch Reduktion wieder zu generieren.

In einer bevorzugten Ausführungsform enthält das NOM zusätzlich zu dem Übergangsmetallkation ein Reduktionsmittel zur Regeneration dieses Metallkations.

In einer Ausführungsform ist das Reduktionsmittel das Radikalfänger-System. So können Substanzen wie Ascorbat, Vitamin oder Glutathion das Übergangsmetallkation höherer Wertigkeit zu einem Kation niedriger Wertigkeit reduzieren.

In einer bevorzugten Ausführungsform werden als Reduktionsmittel die NOD selber verwendet, wobei die NOD unter Einwirkung elektromagnetischer Strahlung das Übergangsmetallkation höherer Wertigkeit zu einem Kation niedriger Wertigkeit reduzieren.

So vermag das Cu²⁺-Kation mit Nitrit oder S-Nitrosothiolen einen Nitrit-Triplett-Komplex zu bilden, wobei unter Bestrahlung mit Licht von 400 bis 470 nm, bevorzugt 400 bis 450 nm, das Nitritanion zu NO₂ oxidiert wird und das Cu²⁺ zu Cu⁺ reduziert wird:

Cu²⁺ + NO₂⁻ → [Cu²⁺ + 3NO²⁻]

[Cu²⁺ + ³NO²⁻] → Cu⁺ + NO₂

Das so erhaltene Cu(I)-Kation kann dann durch Reduktion eines Nitritanions das NO freisetzen, wobei auch hier das NO von hoher Reinheit ist, d.h. eine Reinheit aufweist, wie es für die therapeutische Anwendung vorgeschrieben ist.

Entsprechen stellt die Erfindung in einer besonderen Ausführungsform eine medizinische Auflage bereit, die ein NO-Modul (NOM) umfasst, das photolabile Stickstoffmonoxidvorstufen (NOD) enthält und Cu²⁺-Kationen enthält, wobei durch Einstrahlung von Licht mit einer Wellenlänge zwischen 400 und 470 nm, und bevorzugt zwischen 400 und 450 nm, durch Reduktion der NOD im NOM NO generiert wird, welches aus dem NOM freigesetzt werden kann.

Da bei dieser speziellen Form der Licht-induzierten NO-Freisetzung die Bildung von schädlichen Nebenprodukten unterbleibt, kann auch hier auf das Radikalfänger-System verzichtet werden.

In einer besonderen Ausführungsform ist das Übergangsmetallkation ein Cu²⁺-Ion. Dieses kann durch Licht im Bereich von 400 bis 470 nm (blaues Licht) unter Komplexbildung mit einem Reduktionsmittel zu Cu¹⁺ reduziert werden, wobei das Reduktionsmittel oxidiert wird. Das Cu¹⁺ vermag dann durch eine Redoxreaktion aus den NOD, insbesondere aus dem Nitrit das NO freizusetzen. Entsprechend ist hier die Verwendung von Cu(NO₂)₂ bevorzugt.

Als Reduktionsmittel kann in einer Ausführungsform das Radikalfängersystem verwendet werden.

In einer besonderen Ausführungsform ist das NOM als mehrschichtige Auflage ausgestaltet. Diese mehrschichtige Auflage der vorliegenden Erfindung umfasst (i) eine NOD-haltige Schicht (auch "mittlere Schicht"), in welcher die mindestens eine photolabile NO-Vorstufe (NOD) in gelöster oder suspendierter Form vorliegt, und (ii) eine innere für NO durchlässige Schicht ("innere Schicht"), sowie (iii) gegebenenfalls eine Schutzfolie und/oder eine Rückschicht.

In einer vorteilhaften Ausführungsform umfasst das mehrschichtige NOM zusätzlich eine äußere Schicht ("äußere Schicht"). Die äußere Schicht ist in diesem Zusammenhang eine solche, die sich der mittleren NOD-haltigen Schicht auf der Haut abgewandten Seite direkt oder indirekt anschließt.

Die äußere Schicht kann vorzugsweise zwischen der mittleren Schicht und der Rückschicht angeordnet sein. Gemäß einer Ausführungsform ist die äußere Schicht im Wesentlichen undurchlässig für NO. Sie kann selbstklebend oder nicht-selbstklebend sein. Sollte sie nicht-selbstklebend sein, können Haftmittel vorgesehen werden, um die äußere Schicht mit der Rückschicht klebend zu verbinden.

Im Rahmen der Erfindung ist unter einer Auflage jegliche flächenförmige Vorrichtung zu verstehen, die auf Körperbereiche aufgelegt werden kann. Das Auflegen umfasst hierbei ein einfaches Auflegen ohne engen oder adhäsiven Kontakt, aber auch ein zumindest teilweise adhäsive Verbindung der Auflage mit der Haut. Eine solche adhäsive Verbindung oder Klebeverbindung ist zweckmäßigerweise als reversible Klebeverbindung ausgestaltet.

Die mittlere Schicht der NOM ist dadurch gekennzeichnet, dass sie eine oder mehrere photolabile NO-Vorstufen (NOD) enthält. In bevorzugter Weise enthält sie zudem ein Radikalfänger-System.

Die eingesetzten Substanzen des Radikalfänger-Systems fangen nicht nur die bei der NO-Generierung entstehenden radikalischen Nebenprodukte ab, sondern sorgen dafür, dass die entsprechende Schicht sauerstoffarm oder sogar sauerstofffrei ist und damit auch eine initiale Reaktion des entstandenen NO mit dem Sauerstoff unterbunden wird.

In zweckmäßiger Weise liegen die photolabilen NOD und das Radikalfänger-System in derselben Schicht vor. Dadurch können die bei der Photolyse als Nebenprodukte entstehenden Radikale direkt abgefangen werden, ohne dass sie mit weiteren Substanzen unter Bildung von eventuell toxischen Substanzen abreagieren. Bevorzugterweise sind die NOD und das Radikalfänger-System in der mittleren Schicht enthalten.

In einer alternativen Ausführungsform, beispielsweise bei einer chemischen Inkompatibilität von NOD und Radikalfänger-System, liegen diese beiden Komponenten in unterschiedlichen Schichten vor. Hierbei ist es zweckmäßig, dass das NOD in der mittleren Schicht und das Radikalfänger-System in der inneren Schicht enthalten ist, so dass das durch Photolyse generierte NO mit seinen Nebenprodukten vor dem Auftreten auf die Haut bei dem Durchtritt durch die innere Schicht aufgereinigt wird.

Bei dem "Radikalfänger-System" der vorgenannten Ausführungsformen handelt es sich bevorzugt um ein Antioxidans, und besonders bevorzugt um Ascorbat oder Ascorbinsäure.

Die Konzentration des Radikalfänger-Systems bezogen auf das Gesamtgewicht der sie enthaltenden Schicht(en) kann hierbei bis zu 20 Gew.-% betragen, bevorzugt zwischen 0,25 und 10 Gew.-%, besonders bevorzugt zwischen 3 und 7,5 Gew.-%.

In einer bevorzugten Ausführungsform der Erfindung sind die photolabilen Stickstoffmonoxidvorstufen (NOD) des NOM ausgewählt aus der Gruppe enthaltend organische Nitrate, anorganische Nitrate, Nitrite, Schwefel-, Stickstoff- oder Sauerstoff-Nitrosoverbindungen, NO-Metall-Verbindungen und NO-chelatierende Substanzen.

Photolabile Stickstoffmonoxidvorstufen sind im Stand der Technik bekannt und dem Fachmann geläufig.

Beispiele für photolabile NOD umfassen Diazeniumdiolate (z.B. US Patente Nr. 7,105,502; 7,122,529; 6,673,338), trans[RuCI([15]aneN4)NO]+2, Nitrosyl-Liganden, 6-Nitrobenzo[a]pyrol, S-Nitroso-Glutathion, S-Nitroso-Thiol, Nitroanilin-Derivate (siehe US 2013/0224083), 2-Methyl-2-Nitrosopropan, Imidazoyl-Derivate, Hydroxylnitrosamin, Hydroxylamin und Hydroxyharnstoff.

In einer weiteren Ausführungsform weist das NOM und bevorzugt dessen NOD-haltige Schicht oder Schichten einen Gehalt der NO-Vorstufen von zwischen 0,1 und 50 Gew.-% auf, bevorzugt zwischen 0,25 und 20 Gew.-%, besonders bevorzugt zwischen 0,5 und 10 Gew.-% und insbesondere zwischen 2,5 und 7,5 Gew.-% bezogen auf das Gesamtgewicht der sie enthaltenden Schicht(en).

In bevorzugter Weise handelt es sich bei den NOD um pharmakologisch verträgliche Substanzen. Als solche kommen beispielsweise Nitrite von Alkali- oder Erdalkalimetallen zur Anwendung. Beispielhaft sind hier genannt: LiNO₂, NaNO₂, KNO₂, RbNO₂, CsNO₂, FrNO₂, Be(NO₂)₂, Mg(NO₂)₂, Ca(NO₂)₂, Sr(NO₂)₂, Ba(NO₂)₂, oder Ra(NO₂)₂.

Besonders bevorzugt ist hierbei als NOD das NaNO₂, das in weiterhin bevorzugter Weise zusammen mit Ascorbat oder Askorbinsäure als Radikalfänger-System in der medizinischen Auflage enthalten ist.

Die Konzentration der Nitritsalze bezogen auf das Gesamtgewicht der sie enthaltenden Schicht(en) kann hierbei bis zu 20 Gew.-% betragen, bevorzugt zwischen 0,25 und 10 Gew.-%, besonders bevorzugt zwischen 3 und 7,5 Gew.-%.

In einer weiteren Ausführungsform der Erfindung können die NOD an ein Polymer gekoppelt sein. Entsprechende Methoden zur Kopplung von NOD an Polymere sind beispielsweise durch das US-Patent Nr. 5,405,919 offenbart. In einer Ausführungsform handelt es sich bei dem NOD-gekoppelten Polymer um ein Polymer, das mit Diazoniumdiolatgruppen versehen ist. Ein Beispiel hierfür ist das in der WO 2006/058318 A2 offenbarte, mit Diazoniumdiolatgruppen derivatisierte lineare Polyethylenimin (PEI).

Die Konzentration an erzeugtem NO in dem NOM beträgt zwischen 10 µM und 5 mM, bevorzugt zwischen 100 µM und 3 mM und besonders bevorzugt zwischen 150 µM und 2 mM.

Die Menge an freigesetztem NO beträgt zwischen 50 und 600 ppm und bevorzugt zwischen 160 und 400 ppm. Solche Mengen sind therapeutisch wirksam, ohne zu gravierenden Nebenwirkungen zu führen.

Dem Fachmann sind zahlreiche Systeme bekannt, welche in der Lage sind, mehrfach oxidierte Stickoxide, Sauerstoffradikalanionen, Hydroxylradikale oder aquatisierte Elektronen abzubauen oder zu neutralisieren. Diese wird er entsprechend der jeweiligen Schichtzusammensetzung des NOM auswählen.

Das Radikalfänger-System ist in einer Ausführungsform in der NOD-haltigen Schicht enthalten, so dass es direkt die bei der NO-Bildung entstehenden oxidierten Stickoxide, Sauerstoffradikalanionen, Hydroxylradikale oder aquatisierten Elektronen abbauen oder neutralisieren kann.

Alternativ kann das Radikalfänger-System auch in der inneren Schicht enthalten sein, so dass es beim Durchtritt von NO durch diese zur Haut hin gelegenen Schicht die bei der NO-Bildung entstehenden oxidierten Stickoxide, Sauerstoffradikalanionen, Hydroxylradikale oder aquatisierten Elektronen abbauen oder neutralisieren kann.

Weiterhin besteht die Möglichkeit, dass sowohl die mittlere Schicht als auch die innere Schicht das Radikalfänger-System enthalten.

Für eine lipophile NOM-Schicht, d.h. eine Schicht mit hydrophoben Polymeren, wie es durch ein hydrophobes Polymer bereitgestellt werden kann, eignen sich beispielsweise Antioxidantien wie Tocopherole, Tocotrienole, Tocomonoenole, Irganox®, Irgafos®, Butylhydroxyanisol (BHA) und Butylhydroxytoluol (BHT).

Für eine hydrophile NOM-Schicht, d.h. eine Schicht mit hydrophilen Polymeren, eignen sich insbesondere organische schwefelhaltige Verbindungen wie Glutathion, Cystein, oder Thiomilchsäure oder auch organische Säuren wie Ascorbinsäure, alpha-Liponsäure, Hydroxyzimtsäuren wie p-Cumarsäure, Ferulasäure, Sinapinsäure oder Kaffeesäure, oder Hydroxybenzoesäuren wie Gallussäure, Procatechusäure, Syringasäure oder Vanilinsäure.

Andere bevorzugte Antioxidantien umfassen polyphenolische Verbindungen wie Anthocyane, Flavonoide und Phytoöstrogene.

In bevorzugter Weise enthält das NOM und hierbei bevorzugt die mittlere Schicht darüber hinaus einen oder mehrere der folgenden Stoffe: Katalysatoren, Detergentien, Puffersubstanzen, Chromophore, Substanzen, die die NOD stabilisieren wie bspw. Dimethylsulfoxid oder Ethanol, Substanzen, die die Halbwertszeit von NO erhöhen, wie bspw. in der US 2003/0039697 offenbart, NOD-Stabilisatoren, Antioxidantien, Farbstoffe, pH-Indikatoren, Pflegestoffe, Duftstoffe, pharmakologisch aktive Substanzen.

In einer weiteren bevorzugten Ausführungsform beinhaltet das mehrschichtige NOM und hierbei bevorzugt die mittlere Schicht weiterhin einen Kristallisationsinhibitor. Verschiedene Tenside oder amphiphile Substanzen können als Kristallisationsinhibitoren verwendet werden. Sie sollten pharmazeutisch akzeptabel und für die Verwendung in Medikamenten geprüft sein. Ein besonders bevorzugtes Beispiel für einen solchen Kristallisationsinhibitor ist lösliches Polyvinylpyrrolidon, welches kommerziell erhältlich ist z.B. unter dem Handelsname Kollidon^{®} (Bayer AG). Andere geeignete Kristallisationsinhibitoren beinhalten Copolymere von Polyvinylpyrrolidon und Vinylacetat, Polyethylenglycol, Polypropylenglycol, Glycerol und Fettsäureester von Glycerol oder Copolymere von Ethylen und Vinylacetat.

Optional enthält das NOM einen Penetrationsförderer. Solche Penetrationsförderer (auch "Permeationsenhancer") verbessern die Permeationseigenschaften für das Eindringen der pharmakologisch aktiven Substanzen in die Haut. Beispiele für Penetrationsförder sind u.a. Fettalkohole, Fettsäuren, Fettsäureester, Fettsäureamide, Glycerin oder Glycerin-Fettsäureester, N-Methylpyrrolidon, Terpene wie Limonen, α-Pinen, α-Terpineol, Carvone, Carveol, Limonenoxid, Pinenoxid oder 1,8-Eukalyptol.

Der Fachmann wird in Hinblick auf den jeweiligen Verwendungszweck und basierend auf seinem allgemeinen Fachwissen geeignete Stoffe oder Stoffgemische auswählen. Hierbei wird er vor allem berücksichtigen, dass bei der Verwendung als medizinische Auflage physiologische verträgliche und/oder dermatologisch verträgliche Stoffe und Stoffgemische zur Anwendung kommen.

In einer Ausführungsform der Erfindung enthält die medizinische Auflage und hierbei insbesondere die innere und/oder mittlere Schicht eine oder mehrere pharmakologisch aktive Substanzen. Diese können die pharmakologische Wirkung des NOs unterstützen oder unabhängig von dem NO in einer für die entsprechende Erkrankung therapeutisch relevanten Weise wirken.

In einer Ausführungsform der Erfindung enthält die medizinische Auflage eine oder mehrere der folgenden pharmakologisch aktiven Substanzen: Entzündungshemmer wie bspw. nichtsteroidale Antirheumatika (NSAIDs) oder Corticoide, Immunsuppressiva, Antibiotika, Antikoagulantien, Antithrombotika, antivirale Agenzien, Antimykotika, Lokalanästhetika und Analgetika.

In einer bevorzugten Ausführungsform liegt die pharmakologisch aktive Substanz in Form von wachsartigen Partikeln mit einem niedrigen Schmelzpunkt vor, die bei Kontakt mit der Haut schmelzen und die Substanz freisetzen.

Der pH-Wert der mittleren und/oder inneren Schicht liegt zweckmäßigerweise zwischen 3,0 und 10, bevorzugt zwischen 5,5 und 7,4 und besonders bevorzugt zwischen 6,0 und 7,0.

In einer weiteren Ausführungsform ist das NOM und bevorzugt hierbei die NOD-haltigen Schicht sauerstoffarm oder sauerstofffrei. Entsprechenderweise beträgt der Sauerstoffgehalt des NOM oder der NOD-haltigen Schicht weniger als 20 ppm, bevorzugt weniger als 10 ppm, besonders bevorzugt von weniger als 5 ppm.

Diese erfindungsgemäße Sauerstoffarmut oder Sauerstofffreiheit kann durch Behandeln der Einzelkomponenten des NOM oder Begasen von Zwischenstadien oder des fertigen NOM mit einem inerten Gas (wie Argon oder Stickstoff) hervorgerufen werden. Ein solches NOM ist zweckmäßigerweise gasdicht zu verpacken, so dass die Sauerstoffarmut bzw. Sauerstofffreiheit bis zum Zeitpunkt der Anwendung erhalten bleibt.

In einer weiteren Ausführungsform weist das NOM und bevorzugt hierbei die NOD-haltigen Schicht(en) zur Erzielung der Sauerstoffarmut oder Sauerstofffreiheit einen Sauerstoffabsorber auf. Zu den geeigneten Sauerstoffabsorbern zählen: Irganox®, Irgafos®, Butylhydroxyanisol, Butylhydroxytoluol, Ascorbinsäure oder Pyrogallol.

Die NOD-haltige Schicht weist geeigneterweise ein Flächengewicht von höchstens 70 g/m², vorzugsweise von höchstens 40 g/m² und besonders bevorzugt von höchstens 30 g/m² auf.

Ein geringes Flächengewicht der NOD-haltigen Schicht ist vorteilhaft, da sich das NOM auf diese Weise entsprechend den mikroskopischen Biegebewegungen der Haut verformen kann.

Die innere Schicht weist bevorzugt ein Flächengewicht von 15 bis 55 g/m², vorzugsweise von 15 bis 40 g/m², besonders bevorzugt von 15 bis 30 g/m² und insbesondere von 15 bis 25 g/m² auf.

Die äußere Schicht weist bevorzugt ein Flächengewicht von 5 bis 40 g/m², vorzugsweise von 5 bis 30 g/m², weiter bevorzugt von 5 bis 25 g/m² und besonders bevorzugt von 5 bis 15 g/m² auf.

Ein geringes Flächengewicht der inneren und/oder der äußeren Schicht ist vorteilhaft, da mit abnehmender Schichtdicke auch die Neigung zum kalten Fluss abnimmt. Hierbei ist aber zu berücksichtigen, dass auch die Dicke der inneren Schicht für die ausreichende Verklebung auf der Haut sorgen muss. Daher weist die innere Schicht bevorzugt ein Flächengewicht von mindestens 15 g/m², besonders bevorzugt sogar von mindestens 20 g/m² auf.

In einer Ausführungsform weisen alle Schichten ein identisches Flächengewicht von 20 bis 40 g/m², vorzugsweise von 30 g/m² auf. In einer bevorzugten Ausführungsform beträgt das Flächengewicht der NOD-haltigen Schicht 30 g/m².

In einer besonders bevorzugten Ausführungsform beträgt das Flächengewicht der äußeren Schicht 10 g/m², der mittleren NOD-haltigen Schicht 30 g/m² und der inneren Schicht 20 g/m². Das Flächengewicht beträgt also insgesamt 60 g/m².

In einer Ausführungsform weist das mehrschichtige NOM ohne Rückschicht und Schutzfolie insgesamt ein Flächengewicht von höchstens 120 g/m², vorzugsweise von höchstens 90 g/m², bevorzugt von höchstens 75 g/m² und besonders bevorzugt von höchstens 60 g/m² auf.

Die Schichten des NOM sind bevorzugterweise als flexible Schichten ausgestaltet, so dass sie einen vollflächigen engen Kontakt mit der Haut ausbilden können. Dem Fachmann sind zahlreiche Methoden und Verfahren bekannt, um flexible Schichten herzustellen, so beispielsweise aus der US-Patenten Nr. 6,639,007, Nr. 6,673,871 oder Nr. 7,105,607.

Zweckmäßigerweise weist die dem SEM zugewandte Schicht einen Bereich auf, der für UV-Strahlen durchlässig ist. Dieser stellt damit ein Aktivierungsfenster dar.

In einer weiteren Ausführungsform ist das NOM durch seine Form dem zu behandelnden Körperteil angepasst. So kann es beispielsweise als Strumpf, Socke, Bandage, Stulpe, Handschuh oder Fingerling ausgestaltet sein.

Vorzugsweise sollte daher das NOM aus einem Material aufgebaut sein, das die Eigenschaften des für eine optimale Freisetzung von Stickstoffmonoxid notwendige Energie einer elektromagnetischen Strahlungsquelle nicht beeinflusst oder aufgrund seiner Eigenschaften, die für eine lichtinduzierte Freisetzung von Stickstoffmonoxid notwendigen Lichteigenschaften erst schafft oder optimiert.

Zweckmäßigerweise ist das NOM und hierbei insbesondere die äußere und/oder mittlere Schicht für UV-Strahlung durchlässig. Aufgrund seiner Kenntnis der UV-Durchlässigkeit wird der Fachmann die geeigneten Materialien für das Trägermedium enthaltende Behältnis auswählen. So bietet sich zweckmäßigerweise die Verwendung von UV-durchlässigen Kunststoffen an. Beispiele hierfür sind Polymethylpenten (PMP), modifiziertes Polymethylmethacrylat (PMMA) oder modifiziertes Polyvinylbutyral (Trosivol UV+^{®}).

In einer bevorzugten Ausführungsform ist die innere, der Haut zugewandte Schicht nicht für UV-Strahlung durchlässig, um die Haut vor einer eventuell schädlichen UV-Strahlungsdosis zu schützen.

In einer Ausführungsform enthält die NOD-haltige mittlere Schicht mindestens ein hygroskopisches Polymer oder Copolymer (im Folgenden "hygroskopisches (Co)Polymer"). Bei dem mindestens hygroskopischen (Co)polymer handelt es sich vorzugsweise um Polyvinylpyrrolidon (PVP), Polyvinylalkohol (PVA), Poly(vinylpyrrolidon-co-vinylacetat) oder ein Kohlenhydratpolymer oder um eine Mischung oder ein Copolymer von diesen. Als Kohlenhydratpolymer bevorzugt sind dabei Cellulose oder Derivate davon, Stärke oder Derivate davon, Alginate und Pullulan. Die Cellulose- und Stärkederivate sind vorzugsweise wasserlöslich.

Besonders bevorzugt sind PVP, PVA sowie Mischungen oder Copolymere von diesen. Ganz besonders bevorzugt ist PVP.

Vorzugsweise wird PVP mit einem gewichtsmittleren Molekulargewicht M_{W} von 20.000 bis 3.000.000 g/mol, bevorzugt von 100.000 bis 2.500.000 g/mol, weiter bevorzugt von 500.000 bis 2.000.000 g/mol und besonders bevorzugt von 1.000.000 bis 1.500.000 g/mol verwendet.

Vorzugsweise wird PVA mit einem gewichtsmittleren Molekulargewicht MW von 5.000 bis 100.000 g/mol, bevorzugt von 10.000 bis 50.000 g/mol, weiter bevorzugt von 20.000 bis 40.000 g/mol und besonders bevorzugt von etwa 31.000 g/mol verwendet.

Der mittlere Polymerisationsgrade P_{W} des eingesetzten PVA liegt dabei vorzugsweise zwischen 100 bis 2050, bevorzugt zwischen 200 bis 1025, weiter bevorzugt zwischen 400 bis 825 und besonders bevorzugt bei etwa 630.

Der Hydrolysegrad (Verseifung) des PVA beträgt vorzugsweise 75 bis 100 mol-%, bevorzugt 80 bis 95 mol-%, und weiter bevorzugt 85 bis 90 mol-%.

Eingesetzt werden kann beispielsweise PVP aus der Kollidon-Reihe des Herstellers BASF, insbesondere Kollidon 90 F, sowie PVA, aus der Mowiol-Reihe des Herstellers Clariant, insbesondere Mowiol 4-88.

In einer alternativen Ausführungsform enthält die NOD-haltige mittlere Schicht mindestens ein hydrophobes Polymer. Beispiele für geeignete hydrophobe Polymere sind Polyetrafluorethylen oder Polytrifluorchlorethylen.

Die mittlere Schicht kann beispielsweise im Wesentlichen aus Fasern bestehen, wobei diese als Gewebe oder als Vlies ausgestaltet sein können.

Die innere Schicht ist in einer Ausführungsform als selbstklebende Matrix ausgestaltet. Sie weist vorzugsweise eine ausreichende Löslichkeit und Permeabilität/Durchlässigkeit für das Stickstoffmonoxid auf. Vorzugsweise ist sie undurchlässig für die NOD-Vorstufe.

Als "NO-permeabel" im Sinne der vorliegenden Erfindung wird eine Schicht bezeichnet, welche unter Applikationsbedingungen, also unter Bedingungen auf der Haut des Patienten, für NO durchlässig ist.

Die innere Schicht kann beispielsweise eine oder mehrere NO-permeable Membranen aufweisen. Solche Membranen werden beispielsweise in der US Nr. 2002/0026937 offenbart. In einer bevorzugten Ausführungsform ist die Membran eine selektiv permeable Membran, wie sie beispielsweise durch ein Copolymer aus 70% Polyester und 30% Polyether bereitgestellt wird (e.g. Sipatex™, 10 µm Membran, siehe Hardwick et al., Clinical Science 100: 395-400 (2001)).

Weiterhin kann die innere Schicht auch als NO-permeable Beschichtung auf die mittlere Schicht aufgetragen sein. Derartige NO-permeable Beschichtungen sind aus der US-Anmeldung Nr. 2003/0093143 A oder der US-Anmeldung Nr. 2005/0220838 bekannt.

Die selbstklebende Matrix als innere Schicht kann vorzugsweise ein festes oder semifestes semi-permeables Polymer umfassen, vorzugsweise einen druckempfindlichen Haftmittel (Kleber, PSA) oder eine Mischung solcher Haftmittel. Das (die) druckempfindliche(n) Haftmittel/Kleber bildet/bilden die Matrix, in der ggf. weitere Hilfs- oder Zuschlagsstoffe inkorporiert sind.

Das Haftmittel ist bevorzugt pharmazeutisch akzeptabel in dem Sinne, dass es biokompatibel, nicht sensibilisierend und nicht irritierend gegenüber der Haut ist. Besonders vorteilhafte Haftmittel zur Verwendung in der vorliegenden Erfindung sollten weiterhin die folgenden Anforderungen erfüllen:
1. gleichbleibende Haftmittel und Co-Haftmitteleigenschaften in Gegenwart von Feuchtigkeit oder Transpiration unter üblichen Temperaturschwankungen,
2. gute Kompatibilität mit NO, NOD sowie mit weiteren Hilfsstoffen, die in der Formulierung der Auflage verwendet werden.

Obwohl verschiedene Typen von auf Druck empfindlich reagierenden Haftmitteln in der vorliegenden Erfindung verwendet werden können, ist die Verwendung von hydrophoben Haftmitteln bevorzugt, welche sowohl eine niedrige Wirkstoff- als auch eine niedrige Wasserabsorptionskapazität haben.

In einer Ausführungsform enthält die innere Schicht mindestens ein hydrophobes Polymer. Bei dem mindestens einen hydrophoben Polymer kann es sich bevorzugt um ein Polyisobutylen (PIB) oder ein Gemisch verschiedener Polyisobutylene (PIBs), Polybutylen, Butylkautschuk, eine Styrol-Copolymer, ein Styrol-Butadien-Styrol-Blockcopolymer, ein Styrol-Isopren-Copolymer, Styrol-Isopren, ein Silikonpolymer oder ein Gemisch verschiedener Silikonpolymere, Ethylenvinylacetat-Copolymere (EVA) oder eine Mischung oder ein Copolymer von diesen handeln.

Insbesondere bevorzugt sind ein PIB, ein Gemisch verschiedener PIBs, ein Silikonpolymer sowie ein Gemisch verschiedener Silikonpolymere. Ganz besonders bevorzugt sind ein PIB sowie ein Gemisch verschiedener PIBs.

Gemäß einer Ausführungsform wird ein PIB höheren Molekulargewichts verwendet.

Das PIB höheren Molekulargewichts weist dabei vorzugsweise ein gewichtsmittleres Molekulargewicht M_{W} von 100.000 bis 1.000.000 g/mol, bevorzugt von 150.000 bis 800.000 g/mol, weiter bevorzugt von 200.000 bis 700.000 g/mol und besonders bevorzugt von 250.000 bis 600.000 g/mol auf.

Es kann beispielsweise ein PIB mit einem M_{W} von etwa 250.000 g/mol oder ein PIB mit einem M_{W} von etwa 600.000 g/mol verwendet werden.

Gemäß einer anderen Ausführungsform wird ein Gemisch aus zwei PIBs unterschiedlichen Molekulargewichts verwendet. Vorzugsweise wird dabei ein Gemisch eines PIBs höheren Molekulargewichts mit einem PIB niedrigeren Molekulargewichts verwendet.

Das PIB niedrigeren Molekulargewichts weist dabei vorzugsweise ein gewichtsmittleres Molekulargewicht M_{W} von 10.000 bis 100.000 g/mol, bevorzugt von 20.000 bis 50.000, weiter bevorzugt von 30.000 bis 40.000 und besonders bevorzugt von etwa 36.000 g/mol auf.

Vorteilhafterweise wird diesem Gemisch noch ein niedermolekulares Polybutylen hinzugegeben.

Eingesetzt werden können beispielsweise PIBs aus der Oppanol-Reihe des Herstellers BASF und/oder aus der Durotak-Reihe des Herstellers Henkel. Beispielhaft sind hier Oppanol 10, Oppanol 100, Oppanol 200, Durotak 87-6908 sowie Durotak 618a zu nennen. Die PIBs der Durotak-Reihe können jedoch auch leicht vom Fachmann selbst, z.B. aus denen der Oppanol-Reihe wie B100, B10 etc. angemischt werden.

Vorzugsweise sind die in der inneren Schicht der medizinischen Auflage verwendeten Silikonpolymere von der Art, dass sie ein lösliches polykondensiertes Polydimethylsiloxan (PDMS)/Harznetzwerk bilden, wobei die Hydroxygruppen mit z.B. Trimethylsilyl (TMS)-Gruppen gecapped sind. Vorzugsweise beträgt das Gewichtsverhältnis von Harz zu PDMS 85 : 15 bis 35 : 65, bevorzugt 75 : 25 bis 45 : 55 und besonders bevorzugt 65 : 35 bis 55 : 45. Bevorzugte Silikonpolymere dieser Art sind BIO-PSA druckempfindliche Silikonkleber hergestellt von Dow Corning, insbesondere 07-420x- und 07-430x-Qualitäten, dabei stellt das x einen Herstellerzahlencode dar, der das verwendete Lösungsmittel des jeweiligen Klebers charakterisiert (x =1: Heptan, x = 2: Ethylacetat, x = 3: Toluol). Jedoch können auch andere Silikonkleber verwendet werden. BIO-PSA 07-420x- ist mit einem Harz-PDMS-Gew.-Verhältnis von 65 : 35 von mittlerer Klebrigkeit, BIO-PSA 07-430x hingegen mit einem Verhältnis von 55 : 45 von hoher Klebrigkeit.

In einem weiteren und besonders bevorzugten Aspekt werden zwei oder mehr Silikonkleber als Hauptkleberkomponenten verwendet. Es kann vorteilhaft sein, wenn eine solche Mischung aus Silikonklebern eine Mischung aus stark klebenden, druckempfindlichen Klebern umfassend PDMS mit einem Harz (z.B. 07-430x) und mittelstark klebenden, druckempfindlichen Silikonklebern umfassend PDMS mit einem Harz (z.B. 07-420x), enthält.

Eine solche Mischung, die einen druckempfindlichen Silikonkleber mit hoher und mittlerer Klebrigkeit umfasst, der PDMS mit einem Harz umfasst, ist vorteilhaft, da sie ein optimales Gleichgewicht zwischen guter Adhäsion und geringem kalten Fluss liefert. Ein übermäßiger kalter Fluss kann in einer zu weichen Auflage resultieren, welches leicht an der Verpackung oder an den Kleidungsstücken des Patienten kleben bleibt. Darüber hinaus kann eine solche Mischung ganz besonders nützlich sein, um höhere Plasmaspiegel zu erhalten. Eine Mischung aus dem zuvor genannten 07-420x (mittlere Klebrigkeit) und 07-430x (hohe Klebrigkeit) ist daher insbesondere nützlich für die medizinische Auflage gemäß der vorliegenden Erfindung. Bevorzugt sind hierbei Mischungsverhältnisse zwischen Silikonkleber mit mittlerer Klebrigkeit zu Silikonkleber mit hoher Klebrigkeit von 1:50 bis 50:1, besonders bevorzugt von 1:10 bis 10:1 und insbesondere von 1:1.

Es ist auch möglich, dass die oben genannten hydrophoben Polymere und Copolymere weitere, hydrophile Monomere enthalten, wobei der Anteil dieser hydrophilen Monomore höchstens 50 mol-%, bevorzugt höchstens 30 mol-%, besonders bevorzugt höchstens 10 mol-% beträgt.

In einem weiteren Aspekt der Erfindung werden "SxS-Haftkleber" für die innere Schicht verwendet. SxS Haftkleber sind Styrol-Block-Copolymer-basierte Kleber, die nicht-elastomere Styrolblöcke an den Enden und elastomere Blöcke in der Mitte tragen. Die elastomeren Blöcke können beispielsweise aus Polyethylenbutylen, Polyethylenpropylen, Polybutadien, Polyisobutylen oder Polyisopropen bestehen.

Geeignete SxS-Kleber werden beispielsweise in US 5,559,165 oder US 5,527,536 beschrieben und zeichnen sich aus durch gute Klebeeigenschaften, einfache Herstellung und Verarbeitung sowie gute Hautverträglichkeit.

SxS-Haftkleber können sowohl kommerziell bezogen werden (z.B. als Duro Tak 378-3500 bei National Starch & Chemical) als auch mit einem Heißschmelz Extrusions-Equipment bei der Produktion desmehrschichtigen NOM selbst hergestellt werden. Dazu werden beispielsweise entsprechende Mengen (wenigstens folgender Bestandteile) eines Styrol-Block-Copolymers (z.B. Shell Kraton GX1657 oder Kraton D-1107CU) mit einem aliphatischen und/oder aromatischen Harz (z.B. Keyser Mackay Regalite R1090 oder Regalite R1010 oder Regalite R1100) und einem Öl (z.B. Shell Ondina 933 oder Ondina 941) aus den einzelnen Dosierstationen in den Extruder dosiert, dort vermischt und aufgeschmolzen. Im letzten Schritt wird in den so hergestellten Haftkleber der Wirkstoff in den Extruder eindosiert und die Masse auf Folien laminiert. Typische exemplarische Gewichtsanteile Polymer: Harz: Öl sind z.B. 100:120:20 oder 100:200:50. Durch Variation dieser Mengenanteile können die Eigenschaften des SxS-Haftklebers jeweils an die gewünschten Eigenschaften der medizinischen Auflage (Klebkraft, minimaler Kaltfluss, Klebezeitdauer, Freisetzungsprofil des Wirkstoffes, etc.) angepasst werden.

Vorteilhafte Kombinationen von Polymeren der mittleren und inneren Schicht sind in der nachfolgenden Tabelle aufgeführt.

In dieser Tabelle bedeuten:
"TH-PVA": teilweise hydrolsierter Polyvinylalkohol, Bevorzugte Beispiele für TH-PVAs sind Mowiol 3-85, Mowiol 4-88, Mowiol 5-88, Mowiol 8-88, Mowiol 13-88, Mowiol 18-88, Mowiol 23-88, Mowiol 26-88, Mowiol 32-88, Mowiol 40-88, Mowiol 47-88 und Mowiol 30-92

"VH-PVA": vollständig hydrolsierter Polyvinylalkohol, Bevorzugte Beispiele für TH-PVAs sind Mowiol 4-98, Mowiol 6-98, Mowiol 10-98, Mowiol 20-98, Mowiol 30-98, Mowiol 56-98, Mowiol 15-99 und Mowiol 28-99.

"sol.-PVP" lösliche Polyvinylpyrrolidon-Derivate. Bevorzugte Beispiele für sol-PVAs umfassen Kollidon 12 PF, Kollidon 17 PF, Kollidon 25, Kollidon 30, Kollidon 30 LP und Kollidon 90 F.

"CL-PVP" unlösliche vernetzte (crosslinked) Polyvinylpyrrolidon-Derivate. Bevorzugte Beispiele für CL-PVAs umfassen Kollidon CL, Kollidon CL-F, Kollidon CL-SF und Kollidon CL-M.

"VP/VAc" Kopolymere aus 1-Vinyl-2-Pyrrolidon und Vinylacetat, bevorzugt in einem Massenverhältnis von 6:4. Bevorzugte Beispiele für VP/VAc umfassen Kollidon VA64 und Kollidon VA64 fine.

"SxS Haftkleber" Styrol-Block-Copolymer-basierte Kleber, die nicht-elastomere Styrolblöcke an den Enden und elastomere Blöcke in der Mitte tragen (s.o.).

"Polysaccharide" sind Moleküle bei denen mindestens Monosaccharidmoleküle über eine glycosidische Bindung verbunden sind. Bevorzugte Beispiele umfassen Alginate, Agar-Agar, Carrageen, Guarkernmehl, Konjakgummi, Johannisbrotkernmehl, Hafer-Beta-Glucan, Pektin, Xanthan, Guar Hydroxypropyltrimonium Chlorid und Natrium Hyaluronat.

"Mod. Cellulosen": modifizierte Cellulosen. Bevorzugte Beispiele sind Ethylcellulose (EC), MC (Metolose®, Methylcellulose, Cellulose-methylated), HPMC (Metolose®, MHPC, Hypromellose, Hydroxypropyl Methylcellulose), HPMC-Phthalat (HPMC-P, Hypromellose-Phthalat), AQOAT (HPMC-AS, Hypromellose-Acetat-Succinat), L-HPC (Hydroxypropyl Cellulose, niedrig substituiert), Carboxy Methylcellulose (CMC) und mikrokristalline Cellulose (MCC).

"Standard-Silikonkleber", Silikonpolymer, dass die folgenden drei Klassen umfasst: Silikonkleber mit geringer Klebstärke (low-Tack = Kennung 440X), Silikonkleber mit mittlerer Klebkraft (medium-tack= Kennung 450X) und Silikonkleber mit hoher Klebkraft (high-Tack=Kennung 460X). Ausgewählte Beispiele sind BIO-PSA 7-4401, BIO-PSA 7-4402, BIO-PSA 7-4501, BIO-PSA 7-4502, BIO-PSA 7-4601 und BIO-PSA 7-4602.

"AK-Silikonkleber" Amin-kompatible Silikonkleber, dass die folgenden drei Klassen umfasst: Silikonkleber mit geringer Klebstärke (low-Tack = Kennung 410X), Silikonkleber mit mittlerer Klebkraft (medium-tack= Kennung 420X) und Silikonkleber mit hoher Klebkraft (high-Tack=Kennung 430X). Ausgewählte Beispiele sind BIO-PSA 7-4101, BIO-PSA 7-4102, BIO-PSA 7-4201, BIO-PSA 7-4202, BIO-PSA 7-4301 und BIO-PSA 7-4302.

"HM-Silikonkleber" sog. Hotmelt silikonkleber die lösungsmittelfrei sind und durch Wärmebehandlung flüssig werden.

"PIB": Gemisch eines PIBs mit höherem Molekulargewicht, insb. M_{W} = 250.000 bis 600.000 g/mol, und eines PIBs mit niedrigerem Molekulargewicht, insb. M_{W} = ca. 36.000 g/mol, und vorzugsweise einem niedermolekularen Polybutylen
"BIB", Kopolymer aus Buten und Isobutylen wie bspw. PAR 950.
Polybuten": Thermoplastisches Polymer aus Buten-1, Im Gegensatz zum verzweigt aufgebauten Polyisobutylen sind die Monomere beim PB linear und weitgehend isotaktisch angeordnet, wobei insgesamt hohe Molmassen von 700.000 bis 3.000.000 g/mol erreicht werden. Beispielhaft ist hier Indopol genannt.
"EVA": Copolymer aus Ethylen und Vinylacetat

| **Mittlere Schicht** | **Innere Schicht** |
|---|---|
| TH-PVA | Standard-Silikonkleber |
| VH-PVA | Standard-Silikonkleber |
| Sol. PVP | Standard-Silikonkleber |
| CL-PVP | Standard-Silikonkleber |
| Vp/VAc | Standard-Silikonkleber |
| Polysaccharide | Standard-Silikonkleber |
| Mod. Cellulosen | Standard-Silikonkleber |
| TH-PVA | AK-Silikonkleber |
| VH-PVA | AK-Silikonkleber |
| Sol. PVP | AK-Silikonkleber |
| CL-PVP | AK-Silikonkleber |
| Vp/VAc | AK-Silikonkleber |
| Polysaccharide | AK-Silikonkleber |
| Mod. Cellulosen | AK-Silikonkleber |
| TH-PVA | SxS-Haftkleber |
| VH-PVA | SxS-Haftkleber |
| Sol. PVP | SxS-Haftkleber |
| CL-PVP | SxS-Haftkleber |
| Vp/VAc | SxS-Haftkleber |
| Polysaccharide | SxS-Haftkleber |
| Mod. Cellulosen | SxS-Haftkleber |
| TH-PVA | PIB |
| VH-PVA | PIB |
| Sol. PVP | PIB |
| CL-PVP | PIB |
| Vp/VAc | PIB |
| Polysaccharide | PIB |
| Mod. Cellulosen | PIB |
| TH-PVA | Polybuten |
| VH-PVA | Polybuten |
| Sol. PVP | Polybuten |
| CL-PVP | Polybuten |
| Vp/VAc | Polybuten |
| Polysaccharide | Polybuten |
| Mod. Cellulosen | Polybuten |
| TH-PVA | EVA |
| VH-PVA | EVA |
| Sol. PVP | EVA |
| CL-PVP | EVA |
| Vp/VAc | EVA |
| Polysaccharide | EVA |
| Mod. Cellulosen | EVA |
| TH-PVA | BIB |
| VH-PVA | BIB |
| Sol. PVP | BIB |
| CL-PVP | BIB |
| Vp/VAc | BIB |
| Polysaccharide | BIB |
| Mod. Cellulosen | BIB |

In einer alternativen Ausführungsform ist die innere Schicht nicht adhäsiv ausgestaltet. So ist insbesondere bei offenen oder nässenden Wunden eine nicht-adhäsive Auflage von Vorteil.

In einer weiteren bevorzugten Ausführungsform ist die innere Schicht so ausgestaltet, dass sie die Wundheilung unterstützt, indem sie beispielsweise Wundsekrete aufnimmt oder sogar ein nicht adhäsives Gel mit der Wunde ausbildet.

Entsprechende Wundauflagen sind dem Fachmann bekannt und enthalten beispielsweise Hydrokolloid, Hydrogel, Alginat (bevorzugt Calcium-Alginat) oder Polymerschaum.

Beispiele für solche hydrogelartige Materialien sind anhand folgender Patentanschriften beschrieben:
- CA-A 1 180 622: Gelatine + Polyethylenoxid + Polyethylenimin
- DE-C 28 49 570: Hydrophiles Poly(meth-)acrylsäurederivat in Gegenwart von Polysaccharid /Protein
- DE-C 30 31 304 Basis: hydrophile ethylenisch ungesättige Monomere, vernetzt mit difunktionellen Verbindungen
- EP-B 0 099 758 synthetische Kollagen oder Alginate und andere Biopolymere
- EP-B 0 262 405 Polynatriumacrylat/Polyacrylsäure/Acryloylamid und andere Acrylamidderivate
- EP-B 0 272 074 Copolymere aus ungesättigten, carboxylgruppenhaltigen Monomeren + Di- oder Oligosaccharide
- US-A 3,249,109 Gelatine, Wasser, mehrwertige Alkohole, Pektin
- US-A 4,243,656 Polyacrylatdispersion + Feuchtigkeitsabsorber, Gelatine, Wasser
- WO 2010/046095 A1: Polyurethangelschaum

In einer bevorzugten Ausführungsform ist das Hydrogel aus den folgenden Bestandteilen aufgebaut (siehe DE 3903672 C1):
a) 20 bis 70 Gew.-% wenigstens eines mehrwertigen Alkohols
b) 10 bis 35 Gew.-% wenigstens eines natürlichen Gelierungsmittels (Biopolymer)
c) 0,05 bis 12 Gew.-% wenigstens eines unvernetzten Copolymeren aus einer oder mehreren Vinylcarbonsäuren und ihren Salzen (synthetisches Polymer)
d) 0,05 bis 10 Gew.-% eines Vernetzungsmittels
e) 0 bis 50 Gew.-% Wasser oder physiologische Kochsalzlösung.

Als mehrwertiger Alkohol wird hierbei Glycerin bevorzugt, der allein oder im Gemisch mit weiteren mehrwertigen Alkoholen eingesetzt werden kann. Andere mehrwertige Alkohole sind Ethylenglykol, Diethylenglykol, 1,2-Propandiol, 1,3-Propandiol, 1,2-Butandiol, 1,3-Butandiol, 2,3-Butandiol, 1,4-Butandiol, Glycerinmonoacetat oder ein Gemisch dieser Alkohole. Als natürliches Gelierungsmittel (Biopolymer) wird hierbei in erster Linie Gelatine allein oder im Gemisch mit anderen Biopolymeren, bevorzugt Alginaten, eingesetzt. Besonders bevorzugt wird eine Kombination aus Gelatine und Natriumalginat im Gewichtsverhältnis von 5 : 1 bis 30 : 1. Als weitere Biopolymere, die allein oder im Gemisch mit Gelatine eingesetzt werden, sind Kollagene und Pektine zu nennen. Das als synthetisches Polymer eingesetzte unvernetzte Copolymer ist aus wenigstens einer Vinylcarbonsäure und wenigstens einem ihrer Alkali- oder Ammoniumsalze aufgebaut. Als Vinylcarbonsäuren werden Acrylsäure, Methylcrylsäure und/oder β-Acryloyloxypropionsäure bevorzugt. Andere geeignete Vinylcarbonsäuren sind Vinylessigsäure, Maleinsäure, Fumarsäure, Crotonsäure, Aconitsäure, Itaconsäure und Gemische dieser Säuren. Die erfindungsgemäß verwendeten Vernetzungsmittel sind bevorzugt aus der Gruppe der Metallchelate, Orthotitansäureester, Epoxide, Aziridine, Triazine oder Melamin-Formaldehyd-Harze ausgewählt. Besonders bevorzugt sind hier Aziridine und die Gruppe der Metallchelate, z. B. die Acetylacetonate, z. B. die Übergangsmetallacetylacetonate wie Titan- oder Zirkonacetylacetonat. Das Vernetzungsmittel bewirkt die Vernetzung von Biopolymer mit synthetischem Polymer zu bevorzugt dreidimensionalen Netzwerken.

In einer bevorzugten Ausführungsform ist die innere Schicht ein Hydrokolloid. Was den Begriff "Hydrokolloid", wie er im Rahmen der vorliegenden Erfindung verwendet wird, anbelangt, so ist dieser sehr breit zu verstehen. Im Allgemeinen werden unter Hydrokolloiden zumindest teilweise wasserlösliche, natürliche, aber auch synthetische Polymere verstanden, welche in wässrigen Systemen Gele oder viskose Lösungen bzw. Suspensionen bilden. Dabei handelt es sich üblicherweise um Substanzen, welche den Stoffklassen der Proteine oder Polysaccharide angehören, wobei eine Vielzahl von Hydrokolloiden aus der Natur stammt, insbesondere aus Landpflanzen, Algen, Tieren sowie Bakterien. Hydrokolloide werden oftmals als Verdickungsmittel in Kosmetika und Produkten der Nahrungsmittelindustrie eingesetzt. Für weitergehende Einzelheiten zum Begriff des Hydrokolloids kann insbesondere verwiesen werden auf Römpp Chemielexikon, 10. Auflage, Georg Thieme Verlag, Stuttgart/New York, Stichwort: "Hydrokolloide", Seite 1837, einschließlich der dort referierten Literatur, wobei der diesbezügliche Inhalt durch Bezugnahme hiermit vollumfänglich eingeschlossen ist.

Hierbei ist es besonders vorteilhaft, wenn das Hydrokolloid Gelatine und/oder Kollagen, und insbesondere Kollagen ist.

Bei Kollagen handelt es sich um langfaserige, linearkolloide und hochmolekulare Skleroproteine der extrazellulären Matrix, welche im Bindegewebe, insbesondere in der Haut, im Knorpel sowie in Sehnen, Bändern und Blutgefäßen sowie der proteinhaltigen Grundsubstanz der Knochen von Wirbeltieren, aber auch in phylogenetisch frühen Lebensformen, wie Schwämmen oder Seeanemonen, vorkommen. Die faserige Struktur des Kollagens wird insbesondere durch das Auftreten von Glycin an jeder dritten Position in der Aminosäuresequenz bedingt, da Glycin als sehr raumsparende Aminosäure eine spezielle, helikale Sekundärstruktur in Proteinen bedingt. Die auch als sogenannte Helixbreaker bekannten Aminosäuren Tryptophan und Tyrosin sowie die Disulfidbrücken ausbildende Aminosäure Cystein hingegen liegen in Kollagenen im Allgemeinen nicht vor. Für weitergehende Einzelheiten zum Begriff des Kollagens kann überdies verwiesen werden auf Römpp, Chemielexikon, 10. Auflage, Georg Thieme Verlag, Stuttgart/New York, Stichwort: "Collagene", Seiten 796 und 797, sowie die hierin referierte Literatur, wobei der gesamte diesbezügliche Inhalt hiermit durch Bezugnahme vollumfänglich eingeschlossen ist.

Was speziell die Verwendung von Kollagen im Rahmen der erfindungsgemäßen Wundauflage anbelangt, so ist dieses in der Lage, den Prozess der Wundheilung signifikant zu steigern. Insbesondere besitzt Kollagen eine proteasehemmende Wirkung, was zur Senkung des der Wundheilung abträglichen erhöhten Proteasespiegels im Wundgebiet dient. Ist der Proteasenspiegel im Wundgebiet nämlich erhöht, führt dies oftmals zu einer unkoordinierten Wundheilung und zur Zerstörung von Wachstumsfaktoren, da diese durch Proteasen, wie beispielsweise neutrophile Elastasen oder Matrix-Metallo-Proteasen (MMPs), degradiert werden. Weiterhin regt Kollagen die Bildung von Gefäßstrukturen und Bindegewebe an und unterstutzt so die Wiederherstellung der strukturellen Stabilität des Gewebes. In diesem Sinne kann durch Verwendung von Kollagen als Hydrokolloid die Wundheilung in äußerst effizienter Art und Weise unterstützt werden.

Ähnliche Ausführungen treffen auch auf Gelatine zu, welche ebenfalls in bevorzugter Weise in der Wundauflage als Hydrokolloid eingesetzt werden kann: Unter dem Begriff "Gelatine" wird üblicherweise und im Rahmen der vorliegenden Erfindung ein Polypeptid, welches vornehmlich durch Hydrolyse des in Haut und Knochen von Tieren enthaltenen Kollagens unter sauren oder basischen Bedingungen gewonnen wird, verstanden. Dabei resultiert die Gewinnung von Gelatine unter sauren Bedingungen in der so genannten Typ A-Gelatine bzw. unter alkalischen Bedingungen in der so genannten Typ B-Gelatine. In Wasser, insbesondere unter gleichzeitigem Einfluss von Wärme, quillt Gelatine zunächst stark auf und löst sich darin unter Bildung einer viskosen Lösung, welche schließlich unterhalb von 35°C gallertartig erstarrt. Für weitergehende Einzelheiten zum Begriff der Gelatine kann verwiesen werden auf Römpp Chemielexikon, 10. Auflage, Georg Thieme Verlag Stuttgart/New York, Stichwort: "Gelatine", Seite 1484, sowie die hierin referierte Literatur, wobei der gesamte diesbezügliche Inhalt hiermit durch Bezugnahme vollumfänglich eingeschlossen ist.

Was die Hydrokolloidschicht, insbesondere die Kollagenschicht, weiterhin anbelangt, so kann es erfindungsgemäß vorgesehen sein, dass die Hydrokolloid, vorzugsweise Kollagen, enthaltende Schicht auf einem Hydrokolloidvlies und/oder Hydrokolloidschaum, vorzugsweise einem Kollagenvlies und/oder Kollagenschaum, basiert. In diesem Zusammenhang kann es vorgesehen sein, dass die Hydrokolloidschicht auf Basis von Hydrokolloidvlies und/oder Hydrokolloidschaum, vorzugsweise Kollagenvlies und/oder Kollagenschaum, porcinen, bovinen und/oder equinen Ursprungs, besonders bevorzugt auf Basis von Hydrokolloidvlies und/oder Hydrokolloidschaum, vorzugsweise Kollagenvlies und/oder Kollagenschaum, porcinen Ursprungs, ausgebildet ist.

In erfindungsgemäß besonders bevorzugter Weise kann es vorgesehen sein, dass die das Hydrokolloid, vorzugsweise Kollagen, enthaltende Schicht durch ein Hydrokolloidvlies und/oder Hydrokolloidschaum, vorzugsweise Kollagenvlies und/oder einen Kollagenschaum, insbesondere durch ein Hydrokolloidvlies und/oder Hydrokolloidschaum, vorzugsweise Kollagenvlies und/oder einen Kollagenschaum, porcinen, bovinen und/oder equinen Ursprungs, bevorzugt durch ein Hydrokolloidvlies und/oder Hydrokolloidschaum, vorzugsweise Kollagenvlies und/oder einen Kollagenschaum, porcinen Ursprungs, gebildet ist.

Der Einsatz von Hydrokolloidvlies bzw. Hydrokolloidschaum, vorzugsweise Kollagenvlies bzw. Kollagenschaum, ist gegenüber herkömmlichen Materialien zur Herstellung von Wundauflagen insbesondere mit dem Vorteil verbunden, dass das Material mit dem Wundgrund bzw. der Wundoberfläche nicht verklebt, aber dennoch eine gute Haftung an der Oberfläche erzielt werden kann. Darüber hinaus ist es von besonderem Vorteil, dass Wundauflagen auf Basis von Hydrokolloidschaum bzw. Hydrokolloidvlies, insbesondere Kollagenschaum bzw. Kollagenvlies, keine Fasern bzw. keine festen Bestandteile bzw. Partikel an die Wunde abgeben und somit das Eindringen bzw. ein zusätzliches Herantragen von Fremdkörpern verhindert wird.

Als besonders vorteilhaft hat sich in diesem Zusammenhang erwiesen, wenn die Wundauflage Hydrokolloidschaum, insbesondere Kollagenschaum, d. h. zu einem Schaum verfestigtes bzw. expandiertes Hydrokolloid bzw. Kollagen, aufweist, insbesondere da durch die im Hydrokolloidschaum bzw. Kollagenschaum enthaltenen Poren zudem in effizienter Weise große Mengen Wundsekret aus dem Wundgebiet abfließen können, so dass die Bildung von Staunässe sowie ein zu langer Kontakt von im Wundsekret enthaltenen und der Wundheilung abträglichen Substanzen mit der Wunde selbst verhindert wird. Dabei verhindern die chemischen und physikalischen Eigenschaften von verfestigtem und expandiertem Hydrokolloid bzw. Kollagen (d. h. Hydrokolloid- bzw. Kollagenschaum) dennoch, dass die Wunde austrocknen kann. Darüber hinaus sind derartige Schäume an die Form des Wundgrundes äußerst gut anpassbar, d. h. sie können die Wunde vollflächig bzw. flächendeckend abdecken, ohne dass Aufwölbungen oder dergleichen entstehen. Weiterhin wird unter Verwendung eines Hydrokolloidschaums bzw. eines Kollagenschaums eine besonders gute Gasdurchlässigkeit ermöglicht. Dies ist insbesondere mit dem Vorteil verbunden, dass die Wunde gut mit dem aus in der Wundauflage generierten NO begast wird, was einerseits die physiologischen Wundheilungsprozesse fördert, andererseits auch das Wachstum von Keimen verhindert.

Im Ergebnis wird also durch das Vorsehen der Kolloidschicht bzw. Kollagenschicht einerseits in effizienter Weise Wundsekret abgeführt und andererseits eine gute NO-Gasdurchlässigkeit gewährleistet.

Was die Hydrokolloidschicht, insbesondere die Kollagenschicht, weiterhin anbelangt, so kann es erfindungsgemäß vorgesehen sein, dass diese erhältlich ist durch Aufbringen einer Dispersion oder Lösung eines Hydrokolloids, vorzugsweise eines Kollagens, auf einem Träger und nachfolgende Trocknung, insbesondere Lyophilisation (Gefriertrocknung), vorzugsweise unter Expansion des Hydrokolloids, vorzugsweise des Kollagens. Eine erfindungsgemäß geeignete Hydrokolloid-, vorzugsweise Kollagensuspension oder -lösung, ist insbesondere erhältlich durch Suspendieren bzw. Solubilisieren des Hydrokolloids, insbesondere Kollagens, in Wasser, insbesondere Reinstwasser bzw. in desinfiziertem oder entkeimtem bzw. sterilisiertem Wasser. Dabei kann das Hydrokolloid, insbesondere Kollagen, vorzugsweise in einer Menge im Bereich von 0, 1 bis 5 Gew.-%, insbesondere 0,5 bis 4 Gew. -%, vorzugsweise 0,7 bis 3 Gew. -%, besonders bevorzugt 1 bis 2 Gew.-%, bezogen auf die Hydrokolloidsuspension oder - Lösung, vorzugsweise Kollagensuspension oder -lösung, in der Suspension oder Lösung enthalten sein. Das getrocknete und expandierte Hydrokolloid, vorzugsweise Kollagen, kann schließlich von dem Träger entfernt werden und zur Herstellung der Wundauflage weiterverwendet werden. Zur Gewährleistung der gewünschten Eigenschaften kann das Hydrokolloid bzw. die entsprechende Schicht mit dem Hydrokolloid einen definierten Restfeuchtegehalt aufweisen, was dem Fachmann bekannt ist.

Das Hydrokolloid, vorzugsweise das Kollagen, der Hydrokolloidschicht, insbesondere Kollagenschicht, kann insbesondere porcinen, bovinen und/oder equinen Ursprungs, bevorzugt porcinen Ursprungs, insbesondere aus porciner Haut, sein.

Was die Abmessungen der mindestens ein Hydrokolloid, vorzugsweise Kollagen, enthaltenden Schicht anbelangt, so weist diese vorzugsweise eine Dicke im Bereich von 0,01 bis 100 mm, insbesondere 0,02 bis 50 mm, vorzugsweise 0,05 bis 10 mm, auf. Je nach Schweregrad der zu behandelnden Wunde und Stärke der Wundexsudation ist es vorteilhaft, insbesondere im Falle einer starken Sekretion von Wundwasser (insbesondere z. B. in der exsudativen Phase der Wundheilung), wenn die ein Hydrokolloid, vorzugsweise Kollagen, enthaltende Schicht besonders dick ausgebildet ist. Bei bereits im Heilungsprozess fortgeschrittenen Wunden ist es hingegen meist ausreichend, deutlich dünnere Hydrokolloid- bzw. Kollagenschichten einzusetzen. Erfindungsgemäß ist es somit möglich, die Dicke der Hydrokolloid- bzw. Kollagenschicht an die jeweiligen Erfordernisse anzupassen.

Vorteilhafte Kombinationen von Polymeren der mittleren Schicht und der inneren wundheilungsfördernden Schicht sind in der nachfolgenden Tabelle aufgeführt.

| **Mittlere Schicht** | **Innere Schicht** |
|---|---|
| TH-PVA | Polyurethan(gel)schaum |
| VH-PVA | Polyurethan(gel)schaum |
| Sol. PVP | Polyurethan(gel)schaum |
| CL-PVP | Polyurethan(gel)schaum |
| Vp/VAc | Polyurethan(gel)schaum |
| Polysaccharide | Polyurethan(gel)schaum |
| Mod. Cellulosen | Polyurethan(gel)schaum |
| TH-PVA | Matrix mit Ca-Alginat |
| VH-PVA | Matrix mit Ca-Alginat |
| Sol. PVP | Matrix mit Ca-Alginat |
| CL-PVP | Matrix mit Ca-Alginat |
| Vp/VAc | Matrix mit Ca-Alginat |
| Polysaccharide | Matrix mit Ca-Alginat |
| Mod. Cellulosen | Matrix mit Ca-Alginat |
| TH-PVA | Hydrogel(schaum) |
| VH-PVA | Hydrogel(schaum) |
| Sol. PVP | Hydrogel(schaum) |
| CL-PVP | Hydrogel(schaum) |
| Vp/VAc | Hydrogel(schaum) |
| Polysaccharide | Hydrogel(schaum) |
| Mod. Cellulosen | Hydrogel(schaum) |
| TH-PVA | Hydrokolloid(schaum) |
| VH-PVA | Hydrokolloid(schaum) |
| Sol. PVP | Hydrokolloid(schaum) |
| CL-PVP | Hydrokolloid(schaum) |
| Vp/VAc | Hydrokolloid(schaum) |
| Polysaccharide | Hydrokolloid(schaum) |
| Mod. Cellulosen | Hydrokolloid(schaum) |
| TH-PVA | Suprasorb A^{®} |
| VH-PVA | Suprasorb A^{®} |
| Sol. PVP | Suprasorb A^{®} |
| CL-PVP | Suprasorb A^{®} |
| Vp/VAc | Suprasorb A^{®} |
| Polysaccharide | Suprasorb A^{®} |
| Mod. Cellulosen | Suprasorb A^{®} |
| TH-PVA | Kollagen(schaum) |
| VH-PVA | Kollagen(schaum) |
| Sol. PVP | Kollagen(schaum) |
| CL-PVP | Kollagen(schaum) |
| Vp/VAc | Kollagen(schaum) |
| Polysaccharide | Kollagen(schaum) |
| Mod. Cellulosen | Kollagen(schaum) |
| TH-PVA | Gelatine(schaum) |
| VH-PVA | Gelatine(schaum) |
| Sol. PVP | Gelatine(schaum) |
| CL-PVP | Gelatine(schaum) |
| Vp/VAc | Gelatine(schaum) |
| Polysaccharide | Gelatine(schaum) |
| Mod. Cellulosen | Gelatine(schaum) |

Die in einigen Ausführungsformen vorgesehene äußere Schicht umfasst vorzugsweise mindestens ein hydrophobes Polymer. Bei dem mindestens einen hydrophoben Polymer kann es sich bevorzugt um ein Polyisobutylen (PIB), ein Gemisch verschiedener Polyisobutylene (PIBs), Polybutylen, Butylkautschuk, eine Styrol-Copolymer, ein Styrol-Butadien-Styrol-Blockcopolymer, ein Styrol-Isopren-Copolymer, Styrol-Isopren, ein Silikonpolymer oder ein Gemisch verschiedener Silkonpolymere oder eine Mischung oder ein Copolymer von diesen handeln.

Insbesondere bevorzugt sind ein PIB, ein Gemisch verschiedener PIBs, ein Silikonpolymer sowie ein Gemisch verschiedener Silikonpolymere. Ganz besonders bevorzugt sind ein PIB, sowie ein Gemisch verschiedener PIBs.

Im Übrigen wird für die äußere Schicht auf die Angaben zur inneren Schicht verwiesen. Wie bereits erwähnt können die äußere und die innere Schicht unterschiedliche polymere Zusammensetzungen aufweisen. Vorzugsweise weist die äußere Schicht jedoch die gleiche polymere Zusammensetzung auf wie die innere Schicht.

Das mehrschichtige NOM der vorliegenden Erfindung umfasst ggf. eine Rückschicht, welche inert gegenüber den Komponenten der Matrix ist. Die Rückschicht ist vorzugsweise ein Film, welcher undurchlässig für die Wirkstoffe ist. Solch ein Film kann aus Polyethylenterephthalat (PET), Polyester, Polyamid, Polyethylen, Polypropylen, Polyurethan, Polyvinylchlorid oder einer Kombination aus den zuvor genannten Materialien bestehen. Diese Filme können gegebenenfalls mit einem Aluminiumfilm oder einem Aluminiumdampf beschichtet sein. Die Dicke der Rückschicht kann zwischen 10 und 100 µm, bevorzugt zwischen 15 und 40 µm sein.

Die Rückschicht ist zumindest in einem Teilbereich geeigneterweise durchlässig für die elektromagnetische Strahlung, die im NOM die NOD photolytisch spalten soll.

Das mehrschichtige NOM der vorliegenden Erfindung umfasst ggf. weiterhin eine/ein Schutzschicht/Schutzfolie, welche(s) unmittelbar vor der Verwendung entfernt wird, insbesondere unmittelbar bevor das NOM mit der Haut in Kontakt gebracht wird. Die/das Schutzfolie oder -blatt kann vorzugsweise aus Polyester, Polyethylenterephthalat (PET), Polyethylen oder Polypropylen bestehen, welche(s) gegebenenfalls mit Aluminiumfilm oder Aluminiumdampf oder Fluorpolymeren beschichtet sein kann. Typischerweise liegt die Dicke einer/eines Schutzfolie oder -blatts in den Bereichen zwischen 50 und 150 µm. Um das Entfernen der/des Schutzfolie oder -blatts zu ermöglichen, wenn die medizinische Auflage verwendet werden soll, kann die/das Schutzfolie oder -blatt separate Schutzfolien oder -blätter enthalten, welche überlappende Enden haben, ähnlich wie sie bei der Mehrzahl der konventionellen Pflaster verwendet werden.

In einer besonders bevorzugten Ausführungsform besteht das erfindungsgemäße mehrschichtige NOM aus genau zwei Schichten: einer mittleren NOD-haltigen, und einer inneren Schicht, wobei die innere Schicht direkt an die NOD-haltige Schicht angrenzt. Dieses NOM umfasst des Weiteren ein Rückschicht und eine Schutzschicht.

In einer ebenso bevorzugten Ausführungsform besteht das erfindungsgemäße mehrschichtige NOM aus genau drei Schichten: einer mittleren NOD-haltigen, einer inneren und einer äußeren Schicht, wobei die innere und die äußere Schicht direkt an die NOD-haltige Schicht angrenzen. Diese medizinische Auflage umfasst des Weiteren ein Rückschicht und eine Schutzschicht.

In einer weiteren Ausführungsform der Erfindung ist die medizinische Auflage so gestaltet, dass die Freisetzung von NO in die Umwelt verringert oder gänzlich verhindert.

In einer weiteren Ausführungsform ist die NOM mit einem NO-Sensor gekoppelt, so dass als Rückkopplung auf den gemessenen NO-Wert das Ausmaß der NO-Generierung flexibel angepasst werden kann.

Dieser NO-Sensor als Messvorrichtung zur Quantifizierung des NO kann in einer der Schichten des NOM, also bspw. der Rückschicht, der Schutzfolie, der mittleren Schicht oder der inneren Schicht angebracht sein. Es kann darüber hinaus zwischen der inneren Schicht und der Haut angebracht sein oder auf der Außenseite (d.h. über der Rückschicht bzw. Schutzfolie) der medizinischen Auflage angebracht sein. In einer besonderen Ausführungsform sorgt die NO-Sensor-assoziierte Steuerung dafür, dass bei Überschreiten eines kritischen NO-Wertes das SGE die NO-Generierung gänzlich einstellt.

In einer Ausführungsform der Erfindung wird das NOM so angesteuert, dass in Gehalt an freigesetztem NO über den Zeitraum der Behandlung konstant gehalten wird.

In einer alternativen Ausführungsform der Erfindung wird das NOM so angesteuert, dass der Gehalt an freigesetztem NO über den Zeitraum der Behandlung ansteigt oder abfällt.

In einer Ausführungsform der Erfindung ist das NOM als leicht austauschbarer Verbrauchsartikel ausgestaltet.

In einer weiterhin bevorzugten Ausführungsform ist das NOM so ausgestaltet, dass sie von der Form her eine fehlerfreie Anwendung in der medizinischen Auflage ermöglicht. So ist die Form in bevorzugter Weise als Pflaster oder transdermales System (TTS) ausgestaltet, die nur in einer Orientierung an das SEM befestigbar ist. Dies kann beispielweise über einen Klettverschluss geschehen, der das SEM mit dem NOM verbindet. Darüber hinaus kann das NOM oder das SEM mit einem Arretierungsmechanismus ausgestaltet sein, der nur nach korrekter, d.h. passgenauer Verbindung die Erzeugung und/oder Freisetzung der elektromagnetischen Strahlung seitens des SEM erlaubt. Zweckmäßigerweise kann die medizinische Auflage hierbei mit einem Sensor ausgestattet sein, der eine korrekte Orientierung bzw. Arretierung der von SEM und NOM detektiert und dem Anwender anzeigt.

Die Quelle der elektromagnetischen Strahlung kann dabei eine mit entsprechenden Fluorochromen beschichtete Glüh- oder Gasentladungslampe (niedrigdruck- oder hochdruckentladend), Licht-emittierende Diode (LED), organische Licht-emittierende Diode (OLED), LASER oder jede andere elektromagnetische Strahlungsquelle sein, die in der Lage ist, aus entsprechenden chemischen Vorstufen bzw. Substraten NO zu generieren.

Für eine optimale Spaltung der in dem NOM gelösten oder suspendierten photolabilen NO-Vorstufen kann die Lichtquelle der SEM elektromagnetische Strahlung mit Wellenlängen von 100 bis 2000 nm emittieren oder elektromagnetische Strahlung jeder anderen Wellenlänge emittieren, die allein oder mit Unterstützung chemischer, physikalischer oder biologischer Verfahren eine Spaltung von Stickstoffmonoxid-Vorstufen und dadurch eine Freisetzung von Stickstoffmonoxid induzieren kann.

In einer bevorzugten Weise ist das SEM so mit dem NOM verbunden, dass die beiden über ihre Fläche einen definierten, gleichbleibenden Abstand aufweisen. Dies ist in bevorzugter Weise über ein flexibel gestaltetes SEM zu erreichen, dass auf das NOM aufgelegt werden kann

In einer alternativen Ausgestaltung ist das SEM bei einer flächenförmigen Strahlungsquelle (z.B. durch ein LED-Panel) mit einem Abstandshalter versehen, der somit einen definierten Abstand zu dem NOM herstellen kann.

Um mögliche Verunreinigungen der Umgebungsluft mit NO oder dessen oxidativen Reaktionsprodukten zu mindern, zu vermeiden bzw. zu verhindern, kann die medizinische Auflage über eine Absaugevorrichtung verfügen, die das eventuell austretende schädliche Gase wie NO oder NO₂ absaugt und durch einen Aktivkohlefilter leitet oder durch eine andere Vorrichtung, die in der Lage ist, entsprechende reaktive Gasspezies zu neutralisieren oder zu eliminieren.

Zum Zwecke einer sicheren Anwendung der erfindungsgemäßen Vorrichtung, wird diese über eine elektronisch gesteuerte, anwendungsspezifische Programmwahl inklusive einer Sicherheitsabschaltung der medizinischen Auflage, über entsprechende NO-, NO₂-, Temperatur- und Sicherheitssensorik sowie Fernbedienung und Verbindungsmöglichkeit an externe Steuerungs- und Dokumentationsgeräte bzw. -applikationen verfügen. Zum Sicherheitsmanagement zählt auch eine anwendungs- und benutzerspezifische und elektronisch gesteuerte Erkennung der spezifisch befüllten NOM, wobei diese bevorzugt als auswechselbarer Verbrauchsartikel ausgestaltet sind.

Das mehrschichtige NOM der vorliegenden Erfindung ist dadurch erhältlich, dass
(i) eine Lösung enthaltend NOD und mindestens ein hygroskopisches Polymer oder Copolymer flächig ausgestrichen und getrocknet wird und
(ii) die so erhaltene Schicht mit einer weiteren Schicht sowie mit einer Schicht, welche während der Behandlung für NO durchlässig wird, kaschiert wird.

Vorzugsweise enthält die Lösung in Schritt (i) mindestens ein hydrophiles Lösungsmittel, bevorzugt Ethanol und/oder Wasser.

Im Rahmen der Herstellung des erfindungsgemäßen mehrschichtigen NOM können alle Schichten durch klassische Techniken des Lösens, Mischens, Beschichtens und temperaturgeschützten Trocknens oder auch der nur durch Wärme geprägten Formung erzeugt werden.

Hierzu können nach dem Fachmann lange bekannten Verfahren zunächst Einzelschichten auf bereitgestellten dehäsiv ausgerüsteten Stützfolien, in der Regel aus Polyethylenterephthalat (PET), im lösemittelhaltigen Beschichtungsverfahren mit Rakel-Schlitzdüsen-, Sprüh- oder Rollenauftrag in gleichmäßigen Schichtdicken mit vorzugsweise 15 bis 40 g/m² Auftragsgewicht nach Trocknung für die innere und ggf. 5 bis 40 g/m² für die äußere Schicht sowie höchstens 40 g/m² Auftragsgewicht nach Trocknung für die mittlere NOD-haltige Schicht aufgetragen werden. Im Falle beidseitiger abgestufter Siliconpolymerisierung kann mit dem Substrat direkt in sich gewickelt werden. Anschließend wird der Wirkstoff in einem nichtklebrigen polymeren Hilfsstoff aufgenommen und eine einheitliche Innenphase aus dem wasseraufnehmenden oder wasserquellenden Polymer durch Beschichtung auf bereitgestellten dehäsiv ausgerüsteten Stützfolien im lösemittelhaltigen Beschichtungsverfahren mit Rakel-Schlitzdüsen-, Sprüh- oder Rollenauftrag in gleichmäßigen Schichtdicken mit obigen Werten für das Auftragsgewicht nach Trocknung erzeugt.

Die Herstellung dieser dünnen Schichten ist dem Fachmann heute mit üblichen Beschichtungs-, Trocknungs- und Extrusionsverfahren möglich. Die Zugabe von NOD kann dabei in eine oder mehrere Schichten - mittlere Schicht bzw. Trennschichten-sowohl lösemittelhaltig nach Zwischentrockenprozessen, als auch lösemittelfrei, wenn die NOD bei der Verarbeitungstemperatur flüssig ist oder ein anderes in der Formulierung verbleibendes Lösemittel zugesetzt ist. In jedem Fall ist aufgrund der geringen Diffusionswege eine Verteilung des Wirkstoffs in den Systemkomponenten, falls gewünscht innerhalb von Stunden bis Tagen nach der Herstellung leicht möglich.

Die Schichten können in beliebiger Sequenz hergestellt und nach dem Fachmann bekannten Prozessen aufeinander laminiert werden. Ohne auf dem System bei der Anwendung zu verbleiben, kann eine im Wesentlichen NO-undurchlässige Rückschicht vorgesehen sein, die das NOM vor dem Verkleben mit Textilien schützt. Des Weiteren kann außerdem eine wiederablösbare Schutzschicht vorgesehen sein, die vor der Applikation des NOM auf die Haut entfernt wird.

In einer bevorzugten Ausführungsform erfolgt die Herstellung des erfindungsgemäßen mehrschichtigen NOM mit den in DE 101 47 036 A1 und DE 10 2008 038 595 A1 beschriebenen Verfahren. Mit den dort beschriebenen Verfahren lassen sich mit einer Schutzfolie beschichtete Substrate besonders vorteilhaft beschichten, wodurch ein besonders gleichmäßiger Kleberauftrag erreicht wird.

In alternativen Ausführungsformen können aber auch die folgenden Auftrags- und Laminiersysteme zur Herstellung der erfindungsgemäßen NOM eingesetzt werden:
Knife System - Walzen- und Luftrakel; Double Side System - Doppelstreichmesser; Commabar System - Commabarsystem; Case Knife System - Kastenrakelsystem; Engraved Roller System - Rasterwalzenantrag; 2-Roller System - 2-Walzensystem; 3-Roller System - 3-Walzensystem; Micro Roller System - Mikrowalzensystem; 5-Roller System - 5-Walzensystem; Reverse Roll System; Rotary Screen System - Rotationssiebtechnologie; Dipping System - Foulard/Tauchen; Slot Die System - Düsen/Gießertechnologie; Curtain Coating System; Hotmelt Slot Die System - Hotmelt Breitschlitzdüse; Powder Scattering System - Pulverstreuer.

In einer bevorzugten Ausführungsform wird das erfindungsgemäße NOM durch das sogenannte Slot Die System hergestellt, die auf einer Düsentechnologie basiert. Die Düse stellt hierbei ein geschlossenes Auftragssystem dar, dass aus einer Düsenkammer besteht, in die der zu applizierende Beschichtungsrohstoff hineingepumpt wird. Die Geometrie der Düse, die speziell für jeden Beschichtungsrohstoff hinsichtlich seines Fließbildes ermittelt wird, garantiert einen gleichmäßigen Austritt des Beschichtungsrohstoffs aus dem Austrittsspalt. Eine (Mikro)pumpe fördert das Beschichtungsmedium in hoher Dosiergenauigkeit der Düse zu. Durch die Pumpgeschwindigkeit kann die Beschichtungsmenge präzise definiert werden. Daneben definieren der Austrittsspalt sowie die Warengeschwindigkeit das Auftragsgewicht. Damit sind in Abhängigkeit der Rohstoffviskosität sehr dünne Schichten unter 5 µm möglich.

In einer Ausführungsform der Erfindung wird das NO auf plasmachemischen Wege erzeugt. Neben der Verwendung von "technischen" NO-Gasen für die medizinische Anwendung gibt es Verfahren zur plasmachemischen Erzeugung von Stickstoffmonoxid. Aus den Druckschriften WO 95/07610 A, US 5,396,882 A und DE 198 23 748 A sind Verfahren zur plasmachemischen Erzeugung von NO bekannt, bei dem NO unter Einwirkung einer Glimm-, Funken- oder Lichtbogenentladung in einem Stickstoff (N₂) und Sauerstoff (O₂) enthaltenden Betriebsgas erzeugt wird. Eine Gasentladung der beschriebenen Art führt, wenn sie bei zu niedrigen Temperaturen durchgeführt wird (wie bei der Glimmentladung zu beobachten), zu einer geringen Effizienz der NO-Erzeugung in einem Gasgemisch. Zudem wird unter diesen Bedingungen bevorzugt das für Inhalationszwecke unerwünschte NO₂-Radikal (NO₂^{•}) erzeugt. Um das NO₂-Radikal aus dem Inhalationsgas zu entfernen, ist der Einsatz einer aufwendigen Absorbertechnik erforderlich. Der Nachteil eines Absorbers liegt insbesondere darin, dass das Absorbermaterial häufig ausgetauscht oder wiederaufbereitet werden muss. Die im Vergleich zu einer Glimmentladung energiereichere Funken- oder Lichtbogenentladung bewirkt eine vergleichsweise starke Gasaufheizung, wodurch eine entsprechend effiziente NO-Erzeugung erzielt wird. Die insbesondere am Ansatzpunkt des Funkens hohe thermische Belastung der Elektroden führt jedoch in nachteiliger Weise zu einer starken Elektrodenerosion, d. h. einer fortschreitenden Zersetzung des Elektrodenmaterials. Aufgrund dieser Elektrodenerosion ist das Verfahren einerseits wartungsintensiv, da die Elektroden sehr verschleißanfällig sind. Anderseits muss verhindert werden, dass das im Gas fein verteilte erodierte Elektrodenmaterial zum Patienten gelangt. Dies erfordert eine aufwendige Reinigung des Gases.

Im Rahmen der vorliegenden Erfindung erfolgt die NO-Generierung über Photolyse einer photolabilen Substanz. Danach werden z. B. die in einer nitrithaltigen Lösung (z. B. Natriumnitrit) enthaltenen Nitrit-Ionen (NO₂⁻) mittels elektromagnetischer Strahlung (z. B. UVA-Strahlung mit Wellenlängen zwischen 320 und 440 nm) gespalten (Photolyse), wodurch NO generiert wird. Unter reduktiven Bedingungen bzw. unter Schutzgas (z. B. Stickstoff) verläuft der durch elektromagnetische Strahlung induzierte Zerfall von Nitrit über unterschiedliche, zum Teil auch parallele, thermodynamisch jedoch verschieden gewichtete Kanäle. Es kann angenommen werden, dass im Kanal 1 (Reaktionen 1 bis 5) UVA-Strahlung (mit einem Optimum bei 354 bis 366 nm) Nitrit zum Stickstoffmonoxidradikal (NO^{•}) und zum Sauerstoffradikalanion (O^{•-}) spaltet (Gleichung 1). Das zuletzt genannte Produkt initiiert im Folgenden in wässrigen Lösungen die Bildung des reaktiven Hydroxylradikals (OH^{•}) (Gleichung 2). Das Hydroxylradikal reagiert mit Nitrit, was zur Bildung des Nitrogendioxidradikals (NO₂^{•}) führt (Gleichung 3). Dieses kann mit Stickstoffmonoxid zu Dinitrogentrioxid (N₂O₃) weiterreagieren (Gleichung 4).

NO₂⁻ + hν → NO^{•} + O^{•-} (1)

O^{•-} + H₂O → OH^{•} + OH- (2)

NO₂⁻ + OH^{•} → NO₂^{•} + OH⁻ (3)

NO₂^{•} + NO^{•} → N₂O₃ (4)

N₂O₃ + H₂O → 2 NO₂⁻ + 2 H⁺ (5)

In Kanal 2 (Gleichungen 6-10) scheinen Hydroxylradikale unter den genannten Bedingungen keine Rolle zu spielen, jedoch werden ein "aquatisiertes" Elektron (e⁻_{aq}) sowie ein Stickstoffdioxidradikal gebildet (Gleichung 6). Das Elektron wird bei einem Überschuss an Nitrit auf dieses übertragen und das daraus resultierende Nitritanion (Gleichung 7) wird in Wasser zum NO-Radikal reduziert (Gleichung 8). Die folgenden Reaktionen in Gleichungen (9) und (10) entsprechen denen in Gleichungen (4) und (5). Die Gewichtung von Kanal 1 zum Kanal 2 bildet dabei ein Verhältnis von ca. 40:60.

NO₂⁻ + hv → NO₂^{•} + e⁻_{aq} (6)

e⁻_{aq} + NO₂⁻ → NO₂⁻ (7)

NO₂⁻ + H₂O → NO^{•} + 2 OH- (8)

NO^{•} + NO₂^{•} → N₂O₃ (9)

N₂O₃ + H₂O → 2 NO₂⁻ + 2 H⁺ (10)

Wie aus den Reaktionen 1 bis 10 deutlich wird, wird der photolytische Zerfall von Nitrit von einer parallelen Produktion reaktiver und zytotoxischer, chemischer Spezies begleitet. Aus den Reaktionen in Gleichungen (4) und (9) wird zudem ersichtlich, dass NO₂-Radikale (NO₂^{•}) mit dem in Gleichung (1) gebildeten NO rückreagieren können.

Es wurde erkannt (EP1903003A1), dass durch den Einsatz von mindestens einem System, welches NO₂-Radikale oder Sauerstoffspezies abbaut oder neutralisiert, während der Generierung von Stickstoffmonoxid die Bildung der genannten reaktiven Zwischenprodukte des lichtinduzierten Nitritzerfalls (NO₂^{•}, O^{•-}, OH^{•}, e⁻_{aq}) unterbunden wird oder deren Eliminierung erfolgt, während gleichzeitig die Generierung von Stickstoffmonoxid nicht gemindert wird. Somit wird die Ausbeute frei verfügbarem NO und die Reinheit des Gases erhöht.

Der Anstieg der NO-Freisetzung sowie der hohe Reinheitsgrad beruht auf einer reaktionsbedingten Eliminierung der reaktiven Zwischenprodukte, z. B. gemäß den folgenden Reaktionen (11) bis (17).

N₂O₃ + RS⁻ → NO₂⁻ + RSNO (11)

RSNO + hν → NO^{•} + RS^{•} (12)

NO₂^{•} + RS⁻ → NO₂⁻ + RS^{•} (13)

NO^{•} + RS → RSNO (14)

BA + OH^{•} → BA-OH (15)

VitC + NO₂^{•} → NO₂⁻ + VitC^{•-} (16)

Trol + NO₂^{•} → NO₂⁻ + Trol^{•-} (17)

(Abkürzungen: RS⁻, Thiol; RSNO, S-Nitroso-Thiol; RS^{•}, Thioylradikal; BA, Benzoesäure; VitC, Vitamin C, Askorbat, Askorbinsäure; VitC^{•}, das Radikal von VitC; Trol, Trolox; Trol^{•}, das Radikal von Trolox).

Dieses Verfahren (EP1903003A1) ermöglicht durch die Präsenz dieser oder anderer funktionsgleicher Systeme während der Bildung von Stickstoffmonoxid eine hohe Ausbeute an Stickstoffmonoxid, während gleichzeitig die Bildung von unerwünschten (mehrfach) oxidierten Stickoxiden, insbesondere des NO₂^{•}, sowie von Hydroxylradikalen und reaktiven aquatisierten Elektronen effizient verhindert wird, oder diese Substanzen nach ihrer Entstehung eliminiert werden bzw. nur noch in so geringem Maße erzeugt werden können, dass sie in Lösung verbleiben und nicht in die Gasphase übertreten können. Somit können diese Substanzen z. B. keinen pathologisch relevanten Schaden in Folge der Inhalation der Inhalationsgase verursachen.

Als Systeme, welche reaktive Stickoxidspezies (z. B. Stickstoffdioxid-Radikale oder reaktive Sauerstoffspezies abbauen oder neutralisieren, werden vorzugsweise reaktive Sauerstoffspezies oder Stickoxidspezies (ROS oder RNS)-abbauende bzw. - neutralisierende Substanzen (Antioxidantien) verwendet. Weiter bevorzugt handelt es sich um Askorbinsäure, Askorbat, Vitamin E und seine Derivate, Thiole, andere Antioxidantien, Radikalfänger oder ROS- und RNS-abbauend Enzyme.

Es hat sich darüber hinaus herausgestellt, dass die Bindung oder Eliminierung der genannten reaktiven Zwischenprodukte des lichtinduzierten Nitritzerfalls (NO₂^{•}, O^{•-}, OH^{•}, e⁻_{aq}) auch in einem neutralen pH-Bereich erfolgen kann, wobei aus Nitrit eine maximale NO-Freisetzung in einer maximalen Reinheit erreicht werden kann.

Unter sauren Bedingungen (pH < 7,0) wird in wässrigen Lösungen der "spontane" Nitritzerfall begünstigt. Entsprechend der Gleichungen 18-20 befindet sich das Nitritanion (NO₂⁻) in wässrigen Lösungen in einem Gleichgewicht mit seiner konjugierten Säure, der salpetrigen Säure (HNO₂). HNO₂ seinerseits befindet sich im Gleichgewicht mit Distickstofftrioxid (N₂O₃), welches spontan zu NO^{•} und NO₂^{•} zerfällt.

NO₂⁻ + H⁺ ⇆ HNO₂ (18)

2 HNO₂ ⇆ N₂O₃ + H₂O (19)

N₂O₃ ⇆ NO^{•} + NO₂^{•} (20)

In einer Ausführungsform eines beschriebenen Verfahrens (EP1903003A1) erfolgt damit die UVA-induzierte Generierung von Stickstoffmonoxid vorzugsweise in einem pH-Intervall von 0 bis 12, insbesondere von 1 bis 10, besonders bevorzugt von 1,5 bis 6, speziell von 2 bis 6, ganz speziell von 2,5 bis 4.

Je nach eingesetzter Nitrit- oder Antioxidanskonzentration sowie je nach Höhe des eingesetzten physikalischen Dekompensationsreizes, der zum Zerfall von Nitrit führt, kann durch das erwähnte Verfahren (EP1903003A1) eine hohe Konzentration an Stickstoffmonoxid erreicht werden.

In einer Lösung kann die Menge des generierten Stickstoffmonoxids durch die verwendete Konzentration der das Stickstoffmonoxid freisetzenden Agenzien und durch die physikalische und/oder chemische Induktion, welche für die Freisetzung von Stickstoffmonoxid aus den Agenzien verantwortlich ist, gesteuert werden.

Dabei werden unter dem Begriff "physikalische und/oder chemische Induktion" neben der Intensität der elektromagnetischen Strahlung sowie der Expositionsdauer, welcher die Reaktionslösung ausgesetzt wird, im Allgemeinen auch die Reaktionsparameter verstanden, welche einen Einfluss auf die Bildung von Stickstoffmonoxid an sich und auf die Konzentration an Stickstoffmonoxid haben. Hierzu zählen im Allgemeinen der pH-Wert der Reaktionslösung, der Redoxstatus der Reaktionslösung, die Temperatur der Reaktionslösung, die exponierte Bestrahlungsfläche, die Zeit der Einwirkung einer Induktionsgrößen auf die Stickstoffmonoxid freisetzenden Agenzien, die Entfernung der Quelle der elektromagnetischen Strahlung zur Reaktionslösung, das Spektrum der elektromagnetischen Strahlungsquelle, die Absorptions-, Transmissions-, Reflexionseigenschaften der Reaktionslösung, die Konzentration von biologischen oder chemischen Katalysatoren oder Vermittlersubstanzen, die auch außerhalb der "typischen" physiko-chemischen Bedingungen einer optimalen NO-Freisetzung eine solche aus NO-generierenden Substanzen durch Katalyse oder entsprechende Akzeptoreigenschaften dennoch ermöglichen. Insbesondere sind damit Chromophore und andere Substanzen gemeint, mit deren Hilfe z. B. auch elektromagnetische Strahlung außerhalb des UVA-Spektrumbereiches in der Lage sein könnte aus den entsprechenden NO-bildenden Agenzien eine NO-Freisetzung zu ermöglichen.

So ist es beispielsweise bei konstant gehaltenen Induktionsgrößen durch den Einsatz variierender Konzentrationen der Stickstoffoxid-freisetzenden Substanz(en) möglich, variierende Mengen an Stickstoffmonoxid freizusetzen.

Ferner ist es bei einer konstanten Konzentration der Stickstoffoxid-freisetzenden Substanz(en) möglich, die Freisetzung von Stickstoffmonoxid durch Variation der Einstellparameter der jeweiligen Induktionsgrößen zu verändern. Bei einer konstant gehaltenen Induktionsgröße können daher durch den Einsatz hoher Konzentrationen der NO-freisetzenden Substanzen hohe NO-Mengen freigesetzt werden und umgekehrt. Bei einer konstanten Konzentration der NO-freisetzenden Substanz kann die NO-Generierung durch Variation der Einstellparameter der jeweiligen Induktionsgrößen verändert werden. Die Einstellparameter können dabei alternativ oder gleichzeitig zur Regulation der NO-Erzeugung herangezogen werden. Insbesondere über die gleichzeitige Regelung der NO-Erzeugung über mehrere Einstellparameter kann das Verfahren vorteilhafterweise hinsichtlich der NO-Erzeugung sowie der Erzeugung unerwünschter Nebenprodukte optimiert werden.

Die Substanz, welche zur Freisetzung von Stickstoffmonoxid verwendet und in dem erfindungsgemäßen Verfahren verwendet wird, unterliegt grundsätzlich keiner Beschränkung, solange sie in der Lage ist, unter dem Einfluss von elektromagnetischer Strahlung Stickstoffmonoxid freizusetzen. Sie kann beispielsweise aus der Gruppe bestehend aus
(a) reinen Stoffen oder Stoffgemischen, welche unter dem Einfluss elektromagnetischer Strahlung Stickstoffmonoxid generieren;
(b) Stoffgemische, die zusätzlich zu den unter (a) genannten Stoffen oder Stoffgemischen Hilfssubstanzen enthalten, welche ausgewählt sind aus der Gruppe, bestehend aus Photoakzeptoren, Photoamplifier, Übergangsmetalle, insbesondere Kupferionen, um spontan oder unter physikalischem oder chemischem Einfluss Stickstoffmonoxid generieren; und
(c) Stoffe oder Stoffgemische, die erst als Folge einer zuvor stattgefundenen chemischen Reaktion unter Zuhilfenahme der unter (a) genannten Stoffe und gegebenenfalls der unter (b) genannten Hilfssubstanzen unter dem Einfluss von elektromagnetischer Strahlung spontan oder unter physikalischem oder chemischem Einfluss Stickstoffmonoxid generieren, ausgewählt werden.

Ferner können die unter (a) beschriebenen Substanzen zusätzlich durch Temperaturveränderungen und/oder Feuchtigkeitsveränderungen und/oder Veränderungen des Redoxstatus ihrer Lösungen Stickstoffmonoxid freisetzen.

Die Freisetzung kann ausgehend von wässrigen Nitrit- oder S-Nitrosothiol-Lösungen erfolgen. Dabei ist aus praktischen Gründen die Verwendung einer wässrigen Lösung von Natriumnitrit oder S-Nitrosothiolen als NO-Quelle bevorzugt. Die wässrige Lösung kann eine Konzentration der NO-Vorstufen von vorzugsweise 0,001 bis 10000 mM, insbesondere 0,2 bis 6000 mM, besonders bevorzugt 0,3 bis 5000 mM, speziell 0,4 bis 2000 mM, ganz speziell 0,5 bis 1500 mM, aufweisen.

Die Art der Bestrahlung von NO-generierenden Ausgangssubstraten ist dem auf dem vorliegenden Gebiet tätigen Fachmann an sich bekannt. Es kann jedwede elektromagnetische Strahlung verwendet werden, welche in der Lage ist, photolabile NO-Derivate unter Bildung von Stickstoffmonoxid zu zersetzten. Beispielsweise kann im Rahmen der vorliegenden Erfindung die Herstellung von Stickstoffmonoxid mittels photolytischer Spaltung unter Verwendung einer UVA-Strahlung mit Wellenlängen von beispielsweise 320 bis 440 nm erfolgen. Es kann jedoch auch elektromagnetische Strahlung jeder anderen Wellenlänge verwendet werden, die allein oder unter Zuhilfenahme chemischer, physikalischer oder biologischer Verfahren eine direkte, durch andere Hilfsstoffe induzierte bzw. erleichterte oder katalysierte photolytische Spaltung von NO-generierender NO-Vorstufen (NO-Derivate) induziert.

Die Herstellung von Stickstoffmonoxid kann auch in Lösungen erfolgen, die mit inerten Gasen gesättigt sind. In solchen mit inerten Gasen (Stickstoff (N₂), Helium (H₂), Argon, usw.) gesättigten Lösungen hat das darin gelöste NO eine wesentlich längere Lebensdauer und kann auch in höheren Konzentrationen in Lösung verbleiben. Allgemein wird angenommen, dass die maximale Löslichkeit von NO in wässrigen Lösungen ca. 2 mM beträgt. In diesem Zusammenhang können als wässrige Lösungen auch Kultur- oder Infusions-Medien bzw. -Puffer, Serum, Blut, Gele und alle anderen Stoffe verstanden werden, die in der Lage sind, Gase aufzunehmen.

Das durch Photolyse von photolabilen NO-Vorstufen hergestellte Stickstoffmonoxid kann beispielsweise zu Inhalationszwecken eingesetzt werden. Weitere spezifische Anwendungsgebiete sind die Anregung des Stoffwechsels von Geweben durch äußerliche Anwendung, die strukturelle Modifikation organischer sowie anorganischer Flächen, die Sterilisation oder die Erzeugung von Zytotoxizität. Das durch Photolyse erzeugte Stickstoffmonoxid kann auch zur Begasung von Wunden, insbesondere zur Wundheilung chronischer, nicht-heilender, eventuell Bakterien-befallener Wunden eingesetzt werden. Das Stickstoffmonoxid kann, wenn es in gesättigten Flüssigkeiten erzeugt wird, auch systemisch zur Behandlung von Bluthochdruck eingesetzt werden. Schließlich kann das Stickstoffmonoxid auch in mit Stickstoffmonoxid nitrosierte Träger erzeugt werden, welche spontan wieder NO freisetzen. Das Stickstoffmonoxid kann auch zur Herstellung unterschiedlicher NO-bindender Substanzen (z. B. NO-Donoren) genutzt werden.

Die Qualität eines auch in Lösungen gelagerten bzw. eingebrachten Gases für medizinische Anwendungen muss hohen Anforderungen genügen. Schon eine geringe Verunreinigung des Gases führt zur Bildung unerwünschter und eventuell giftiger Nebenprodukte. Die Entstehung dieser Nebenprodukte bei längerer Lagerung von Stickstoffmonoxid-enthaltenden Gasflaschen sowie bei der plasmatechnischen Herstellung von Stickstoffmonoxid und die Entfernung dieser Radikale stellen einen großen technischen sowie finanziellen Nachteil dar. Die Vorteile des photolytischen Verfahrens zur Herstellung von Stickstoffmonoxid-haltigen Lösungen sind die Einfachheit der Herstellungsmethode des NO-haltigen Gases, der besonders hohe Reinheitsgrad des hergestellten NO-Gasgemisches, geringe Folgekosten und keine Lagerungskosten, die besonders einfache Handhabung der NO-Erzeugung sowie der Reinheitskontrolle, das unvergleichlich günstige Verhältnis der Herstellungskosten zu der Menge des hergestellten NO-Gases.

### Erfindungsmäßige Vorrichtung

Die vorliegende erfindungsgemäße Vorrichtung ist eine aus mindestens zwei Lagen modular aufgebaute Auflage, die in der Lage ist, mittels der Emission elektromagnetischer Strahlung eines Lichtmoduls, in einem dazu enganliegenden Absorptionsmodul eingelagerte photolabile Stickstoffmonoxid-Vorstufen zu spalten, sodass photolytisch Stickstoffmonoxid erzeugt werden kann, welches zur Unterstützung medizinischer Therapien bei Tier und Mensch sowie zur Generierung von NO verwendet werden kann.

Die Vorteile einer solchen Vorrichtung liegen auf der Hand. Aufgrund des limitierten Lösungsverhaltens von NO können in dem Absorptionsmodul zwar physiologisch relevante NO Konzentrationen generiert werden, die jedoch weit unter denen liegen, die für den Menschen gesundheitliche Schäden verursachen könnten. Ferner kann durch einen direkten Kontakt der menschlichen Körperoberfläche mit dem photolytisch erzeugten Stickstoffmonoxid-freisetzenden Modul der Vorrichtung eine wesentlich genauere NO-Behandlung erreicht werden als z. B. mit NO-haltigen Gasgemischen bzw. spontan zerfallenden NO-Donoren. Weiterhin stellt die Möglichkeit, dass die Vorrichtung je nach Beladung mit den entsprechenden NO-Vorstufe von unterschiedlichen Endverbrauchern benutzt werden kann, von Laien bis hin zum Fachmann, den wesentlichen Vorteil der erfindungsmäßigen Vorrichtung gegenüber anderen NObasierten Therapien dar.

Die erfindungsmäßige Vorrichtung besteht mindestens aus einem elektromagnetische Strahlung-emittierenden Modul (im Rahmen der Erfindung als SEM = Strahlung-emittierendes Modul bezeichnet) sowie einem dazu enganliegenden, die photolabilen Stickstoffmonoxid-Vorstufen-enthaltenden Modul (im Rahmen der Erfindung als NOM = NO-Modul bezeichnet).

Das SEM generiert in dem NOM Stickstoffmonoxid durch photolytische Spaltung der darin eingelagerten photo- oder redox--labilen Stickstoffmonoxidvorstufen. Dabei ist das NOM ein integraler, fest mit dem SEM verbundener Bestandteil der Gesamtvorrichtung. Alternativ kann das SEM und das NOM auch voneinander getrennt genutzt werden und zwar in dem Sinne, dass die Bestrahlung des NOM nicht in direktem aufgelegten Kontakt zum SEM erfolgt, sondern mit einem räumlichen Abstand zu diesem.

Wichtig ist, dass die Lichtdurchflutung des NOM mitsamt den darin eingelagerten, das Stickstoffmonoxid freisetzenden Reaktionssubstanzen im Sinne eines induzierten Stoffzerfalls bzw. einer Freisetzung von Stickstoffmonoxid optimal bzw. maximal ist. Für eine optimale Spaltung der in dem NOM eingelagerten photolabilen NO-Vorstufen kann das SEM elektromagnetische Strahlung mit Wellenlängen von 100 bis 2000 nm emittieren oder elektromagnetische Strahlung jeder anderen Wellenlänge, die allein oder mit Unterstützung chemischer, physikalischer oder biologischer Verfahren eine Spaltung von Stickstoffmonoxid-Vorstufen induzieren und dadurch eine Freisetzung von Stickstoffmonoxid generieren kann. Die Quelle der elektromagnetischen Strahlung kann dabei eine mit entsprechenden Fluorochromen beschichtete Glüh- oder Gasentladungslampe (niedrigdruck- oder hochdruckentladend), Licht-emittierende Diode (LED), organische Licht-emittierende Diode (OLED), LASER oder jede andere elektromagnetische Strahlungsquelle sein, die in der Lage ist, aus entsprechenden chemischen Vorstufen bzw. Substraten NO zu generieren.

Die Quelle der elektromagnetischen Strahlung der SEM, die in direktem Kontakt oder auch aus einiger Entfernung die NO-Freisetzung im NOM induziert, kann mit der elektronischen Steuerungseinheit der Lichtquelle räumlich kompakt in einem Teil/Gehäuse verbaut sein oder von dieser Steuerungseinheit räumlich getrennt und lediglich über eine Drahtverbindung verbunden sein oder gar gänzlich getrennt sein, wobei in diesem Fall die Steuerung der Lichtquelle durch die Steuerungseinheit ferngesteuert stattfinden kann.

Das NOM sollte aus einem Material/Stoff/Träger/Medium aufgebaut sein, das die Eigenschaften des für eine optimale Freisetzung von Stickstoffmonoxid notwendige Energie einer elektromagnetischen Strahlungsquelle nicht beeinflusst oder aufgrund seiner Eigenschaften, die für eine lichtinduzierte Freisetzung von Stickstoffmonoxid notwendigen Lichteigenschaften erst schafft oder optimiert. Während das SEM als konstanter, sich nicht verbrauchender Teil der Vorrichtung anzusehen ist, ist das NOM als ein leicht aus tauschbarer bzw. auswechselbarer Verbrauchsartikel der Vorrichtung anzusehen. Dabei ist das NOM als Trägermedium anzusehen, welches vorzugsweise chemisch stabile oder stabilisierte, potentiell NO- speichernde und somit potentiell wieder NO-freisetzende Substanzen (z. B. organische oder anorganische Nitrate, Nitrite, S-, N-oder O-Nitrosoverbindungen, NO-Metall-Verbindungen, NO-chelatierende Substanzen), allein oder in unterschiedlichen Kombinationen enthält, welche in reiner Form oder gelöst in unterschiedlichen Lösungsmitteln, in einer katalysierten, z.B. durch Ionen von Übergangsmetallen, oder nicht-katalysierten, physikalisch gestarteten und/oder chemischen Reaktion aus dem NOM Stickstoffmonoxid freisetzen können.

Das die potentiell wieder NO-freisetzende Substanzen enthaltende Material/Medium der NOM kann eine mehr oder minder viskose bzw. dünn- oder dickflüssige Lösung/Flüssigkeit sein, ein Gel, eine Folie, ein Film, Schaum, Textil, Vlies, Kunststoff, Naturstoff oder ein Trägermedium jeder anderen Stoffklasse, welche in der Lage ist oder in eine solche gebracht werden kann NO-freisetzende Substanzen oder deren stabile Vorstufen zu speichern bzw. zu tragen und NO zu generieren bzw. freizusetzen.

Der Vorteil der Verwendung eines auswechselbaren bzw. austauschbaren NOM ist, dass durch die Befüllung eines NOM mit reaktiven Agenzien in unterschiedlichen Kombinationen und Konzentrationen unterschiedliche, charakteristische, benutzungs- und behandlungsspezifische NO-Freisetzungsmuster in bzw. aus dem NOM generiert werden können. Somit kann erreicht werden, dass die durch die Vorrichtung generierten NO-Freisetzungsmuster durch die Wahl eines spezifisch bestückten NOM eine an die Fach- und Verantwortungskompetenz des Endanwenders angepasste Anwendungsoptimierung ermöglicht. Was die Befüllung des NOM betrifft so werden dort Mengen von vorzugsweise 0,001 bis 10000 mM, insbesondere 0,01 bis 6000 mM, besonders bevorzugt 0,1 bis 5000 mM, speziell 0,4 bis 2000 mM, ganz speziell 0,5 bis 1500 mM der jeweiligen oder kombinierten NO-Vorstufen (z. B. Nitrit oder S-Nitrosothiole) gewählt.

Die NO-Erzeugung in bzw. aus dem NOM der erfindungsgemäßen Vorrichtung wird vorzugsweise anhand der Manipulation verschiedener Einstellparameter an dem SEM oder auch NOM geregelt. Dabei gelten als Einstellparameter die eingesetzten Konzentration der NO-freisetzenden Agenzien, die Stärke der elektromagnetischen Strahlung und die Eigenschaften der weiteren physikalischen und/oder chemischen Induktionsgrößen, welche für die NO-Freisetzung aus den Agenzien verantwortlich sind. Ferner können als mögliche Induktionsgrößen einer NO-Freisetzung aus potentiell NO-generierenden Substanzen einzeln oder in unterschiedlichen Kombinationen folgende Parameter variiert und genutzt werden:
- der pH-Wert,
- der Redoxstatus (Präsenz reduzierender oder oxidierender Stoffe),
- die Temperatur,
- Stromfluss und/oder Spannung,
- der umgebener Druck,
- die Intensität der elektromagnetischen Strahlung und Expositionsdauer, welcher die NOD in dem NOM ausgesetzt wird,
- die exponierte Bestrahlungsfläche,
- die Zeit der Einwirkung einer Induktionsgröße auf die NO-freisetzenden Agenzien,
- die Entfernung der Quelle der elektromagnetischen Strahlung zur Reaktionslösung,
- das Spektrum der elektromagnetischen Strahlungsquelle,
- die Absorptions-, Transmissions-, Reflexionseigenschaften der NOM-Schichten,
- oder die Konzentration von biologischen oder chemischen Katalysatoren oder Vermittlersubstanzen, die auch außerhalb der "typischen" physiko-chemischen Bedingungen einer optimalen NO-Freisetzung eine solche aus NO-generierenden Substanzen durch Katalyse oder entsprechende Akzeptoreigenschaften ermöglichen (z. B. mit Hilfe von Chromophoren und anderen Substanzen, mit deren Hilfe z. B. auch elektromagnetische Strahlung außerhalb des UVA-Spektrumbereiches in der Lage sein könnte, aus den entsprechenden NO-bildenden Agenzien eine NO-Freisetzung zu ermöglichen).

In Bezug auf den zuletzt genannten Punkt sei darauf hingewiesen, dass insbesondere in der Anwesenheit von Ionen von Übergangsmetallen, z. B. Cu²⁺, Nitritlösungen bei wesentlich längeren Wellenlängen absorbieren können als es reine Nitritlösungen tun und somit das Nitrition auch durch Licht in Wellenlängen von 400 - 450 nm unter NO-Freisetzung gespalten werden kann.

Was die weiter oben angesprochenen Stellgrößen betrifft, so könnte bei der erfindungsgemäßen Vorrichtung bei einer konstant gehaltenen Induktionsgröße, durch den Einsatz variierender Konzentrationen der Stickstoffoxid freisetzenden Substanzen variierende Mengen an Stickstoffmonoxid erzeugt werden. Andererseits könnte bei einer konstanten Konzentration der Stickstoffmonoxid freisetzenden Substanz, die Freisetzung von Stickstoffmonoxid in dem Trägermedium durch Variation der Einstellparameter der jeweiligen Induktionsgröße verändert werden.

Die erfindungsgemäße Vorrichtung verfügt über eine zuverlässige sicherheits- und behandlungsrelevante Sensorik (z. B. für NO, NO₂, Temperatur, Lichtstärke, Hautrötung, Zeitabschaltung, usw.) sowie über Anbindungs- und Verbindungsmöglichkeiten zu externen Geräten, wie z. B. Computer, Smartphones, usw.). Zudem können alle Funktionen der Vorrichtung direkt oder über Software-gesteuerte Applikationen ferngesteuert werden und zudem kann die Vorrichtung mit allen externen Geräten im Sinne einer Feedbackregulation "kommunizieren".

Das mit Hilfe der hier beschriebenen erfindungsgemäßen Vorrichtung erzeugte NO kann zur Anregung des Stoffwechsels von Geweben durch äußerliche Anwendung, im Bereich der Dermatologie zur Behandlung von chirurgischen oder unfallbedingten Wunden, chronischen, nicht- bzw. schlechtheilenden und/oder bakteriell bzw. Pilz-befallenen Wunden sowie zur Behandlung von dermatologischen Erkrankungen aus dem Formenkreis der entzündlichen, immunologisch gesteuerten bzw. Autoimmunerkrankungen verwendet werden. Beispiele für mögliche Anwendungsgebiete wären:
- Behandlung diabetischer Füße und Wunden,
- Behandlung neuropathischer Schmerzen beim Diabetes und anderen Erkrankungen,
- Behandlung von Krampfadern,
- Behandlung lokaler oberflächlicher als auch tiefsitzender Ischämien und thrombopathischer Erkrankungen von Geweben,
- akute und chronische Entzündungen der Haut,
- Allergien der Haut,
- parasitäre Infektion der Haut,
- atopische Dermatitis insbesondere Neurodermitis,
- Dermatomyositis,
- Pemphigus vulgaris und/oder andere lokale und systemische Infektionen und/oder akute und chronische Entzündungssituationen,
- Wunddefekte, wie der chronische diabetisch-neuropathische Ulcus,
- Ulcus cruris,
- Dekubituswunden,
- sekundär heilende infizierte Wunden,
- reizlose, primär heilende Wunden, wie insbesondere ablative Riss- oder Schürfwunden,
- (Haut-)Transplantate,
- Behandlung des diabetischen Schmerzes der unteren Extremitäten (Fuß oder Bein); und
   Behandlung von schlecht perfundierten Lappenplastiken.

Zudem könnte es möglich sein, durch Behandlung größerer Körperareale auch systemische Erkrankungen, wie z. B. den erhöhten Blutdruck (Hypertonie) und verwandte hämodynamische Erkrankungen zu adressieren.

Zum Zweck einer Behandlung wird das NOM auf das zu exponierende Areal aufgelegt und vorzugsweise im direkten Kontakt oder auch aus einer Entfernung der aus dem SEM emittierten elektromagnetischen Strahlung ausgesetzt. Die Behandlungszeit kann zwischen wenigen Sekunden und vielen Stunden dauern.

In einer bevorzugten Ausführungsform der Erfindung beträgt die Behandlungszeit zwischen 5 bis 30 Minuten, bevorzugt zwischen 7,5 bis 20 Minuten und besonders bevorzugt zwischen 10 bis 15 Minuten.

In einer Ausführungsform der Erfindung wird die medizinische Auflage zur Behandlung von Erkrankungen verwendet. Hierbei wird in zweckmäßiger Weise die medizinische Auflage auf den zu exponierenden Areal des Körpers, also bspw. einen Rumpfabschnitt, oder einen Extremitätenabschnitt aufgelegt und dann durch die UV-induzierte Freisetzung oder einer durch eine Redoxreaktion hervorgerufene Freisetzung von NO aus dem NOM dieses entsprechende Areal dem NO ausgesetzt.

Die erfindungsgemäße medizinische Auflage kann somit nicht nur zur Behandlung von chronischen oder akuten Erkrankungen eingesetzt werden, sondern auch zur möglichen Prävention solcher Erkrankungen. Sofern nicht anders ausgeführt, umfasst der Begriff "Therapie" oder "Behandlung" alle Maßnahmen zur Linderung, Heilung oder Prävention der hier relevanten Erkrankungen.

Ein derartiges Auflagebehandlung kann in Abstanden von 1, 2, 3, 4, 5, 6, oder 7 Tagen oder sogar mehrmals täglich angewendet werden, wobei eine 2 bis 3-malige tägliche Anwendung bevorzugt ist.

Hierbei verbleibt das NOM in zweckmäßiger Weise auf der Haut und kann durch Aufbringen des SEM für eine bemessene Zeit wieder zur NO-Generierung und NO-Freisetzung angeregt werden. Dies wird durch einen "Überschuss" an NOD im NOM ermöglicht, die mehrfache Bestrahlungsintervale erlauben.

Zur Steuerung der Behandlungsdauer kann die medizinische Auflage in bevorzugter Weise eine Zeitsteuerungseinheit beinhalten, die nach einer fest vorgegebenen oder bevorzugt flexibel programmierbaren Zeit die Strahlungsquelle des SEM abschaltet und damit die NO-Generierung unterbindet.

Darüber hinaus kann die medizinische Auflage einen Farbstoff enthalten, der nach einer bestimmten Zeit eine Farbveränderung erfährt, so dass der Benutzer über das Ende des Behandlungszeitraums informiert ist.

Weiterhin kann die medizinische Auflage auch eine Vorrichtung zur Messung der Durchblutung umfassen, der anhand des Therapieerfolges eine besonders gute Steuerung der Behandlungsdauer und/oder Behandlungsintensität erlaubt. Dem Fachmann sind zahlreiche Vorrichtungen zur Messung der Durchblutung bekannt. Beispiele hierfür sind Gefäßtachometer, oder der in der WO 97/46853 offenbarte Mikrosensor. Dieser Sensor umfasst einen Indikator-durchlässigen Einsatz, der in einer Öffnung eines Indikator-Behälters, der durch ein Behältnis gebildet ist, angeordnet ist, wodurch der Einsatz einen durchlässigen Wand-Abschnitt des Behälters bildet.

Als Surrogatparameter für die Hautdurchblutung können weitere vaskulär bedingte Messparameter wie die Rötung der Haut oder die Hauttemperatur dienen, für die entsprechende Messmethoden und -geräte aus dem Stand der Technik bekannt sind.

In einem weiteren Aspekt stellt die Erfindung ein Verfahren zur Behandlung eines Patienten bereit, das die folgenden Schritte umfasst:
a. Auflegen oder Aufkleben einer medizinischen Auflage gemäß der Erfindung auf den zu behandelnden Körperabschnitt; und
b. Generierung und Freisetzung von NO durch Einschalten der UV-Strahlungsquelle der SEM.

In einer bevorzugten Ausführungsform ist bei diesem Verfahren die Behandlung ausgewählt aus der Gruppe enthaltend:
- Anregung des Stoffwechsels von Geweben durch äußerliche Anwendung bei Mensch und Tier;
- Behandlung von chirurgischen oder unfallbedingten Wunden;
- Behandlung von chronischen, nicht- oder schlecht-heilenden Wunden;
- Behandlung von bakteriell und/oder durch Pilze befallenen Wunden;
- Behandlung von dermatologischen Erkrankungen aus dem Formenkreis der entzündlichen, immunologisch gesteuerten oder Autoimmunerkrankungen;
- Behandlung diabetischer Füße und Wunden;
- Behandlung neuropathischer Schmerzen;
- Behandlung von Krampfadern;
- Behandlung von lokalen oberflächlichen oder tiefsitzenden Ischämien und thrombopathischer Erkrankungen von Geweben;
- Behandlung akuter und chronischer Entzündungen der Haut;
- Behandlung von Allergien der Haut;
- Behandlung von parasitären Infektionen der Haut;
- Behandlung der atopischen Dermatitis insbesondere Neurodermitis, Dermatomyositis und Pemphigus vulgaris;
- Behandlung von Wunddefekten, wie dem chronischen diabetischneuropathischen Ulcus, Ulcus cruris, Dekubituswunden;
- Behandlung größerer Körperareale zur Therapie systemischer Erkrankungen wie z. B. des erhöhten Blutdrucks (Hypertonie) und verwandten hämodynamischen Erkrankungen;
- Behandlung von Patienten mit (Haut)transplantaten;
- Behandlung des diabetischen Schmerzes der unteren Extremitäten (Fuß oder Bein); und
- Behandlung von schlecht perfundierten Lappenplastiken.

In einer bevorzugten Ausführungsform wird das Verfahren zur Behandlung chronischer Wunden der unteren Extremitäten von Diabetikern angewendet.

Zweckmäßigerweise ist das erfindungsgemäße Verfahren dadurch gekennzeichnet, dass die Behandlung durch Auflegen oder Aufkleben der medizinischen Auflage auf einen Rumpfabschnitt oder Extremitätenabschnitt mit UV-induzierter NO-Freisetzung erfolgt. Eine solche Behandlung kann hierbei zwischen wenigen Sekunden und vielen Stunden dauern.

Bevorzugterweise dauert die Behandlung durch UV-induzierte NO-Freisetzung zwischen 5 bis 30 Minuten, bevorzugt zwischen 7,5 bis 20 Minuten und besonders bevorzugt zwischen 10 bis 15 Minuten.

In einer besonders bevorzugten Ausführungsform wird die erfindungsgemäße medizinische Auflage zur Behandlung chronischer Wunden der unteren Extremitäten und hierbei insbesondere bei Diabetikern eingesetzt. Hierbei kann zudem durch die Behandlung im Sinne einer Prophylaxe das Entstehungsrisiko chronischer Wunden sowie die Anzahl medizinischer Amputationen verringert werden. Dadurch gehen die Reduktion der neuropathischen Beinschmerzen und die Herstellung eines verbesserten Wundmilieus mit einer spürbar verbesserten Lebensqualität der Patienten einher. Darüber hinaus ist durch eine Verkürzung der Wundversorgung eine signifikante Minderung der Behandlungskosten zu erwarten.

In einer Ausführungsform der Erfindung wird die medizinische Auflage zur Therapie schlechtheilender Wunden eingesetzt. Eine gestörte arterielle Durchblutung und/oder venöse Rückflussstörungen sind maßgebliche Ursachen in der Entstehung sowie Chronizität von Wunden der unteren Extremitäten. Eine NO-bedingte arterielle Vasodilatation verbessert die Durchblutung des betroffenen Gewebes und durch die antithrombogene Wirkung des NO wird ein venöser Rückfluss des Blutes wesentlich gefördert bzw. erleichtert. Die NO-abhängige Verbesserung beider hämodynamischer Parameter stellt den entscheidenden therapierelevanten Aspekt einer lokalen sowie systemischen Wirkung, die das Risiko der Entstehung von Wunden signifikant mindert bzw. deren Heilung wesentlich beschleunigt. Das mittels der medizinischen Auflage dem Körper in lokal begrenzter Form dem zu behandelnden Extremitätenabschnitt oder dem Rumpfabschnitt zugeführte NO kann daher erfolgreich zur Therapie schlechtheilender Wunden angewendet werden.

In einer besonderen Ausführungsform wird die erfindungsgemäße medizinische Auflage zur Behandlung des diabetischen Schmerz der unteren Extremitäten, also von Fuß und/oder Bein, eingesetzt. Der diabetische Schmerz ist ein sehr häufiges Ereignis im Verlauf einer Diabeteserkrankung. Der diabetischer Fuß-/Beinschmerz ist ein Ergebnis langandauernder erhöhter Blutglukosekonzentrationen, die die Grundursache für die während einer Diabeteserkrankung beobachteten Nerven- sowie Gefäßschädigung ist. Eine NO-bedingte arterielle Vasodilatation verbessert die Durchblutung des betroffenen Gewebes und hilft die Schmerzweiterleitung im Sinne einer Schmerzminderung zu beeinflussen. Das mit der medizinischen Auflage von außen dem Fuß und/oder Bein zugeführte NO kann daher erfolgreich zur Therapie des diabetischen Fuß-/Beinschmerz angewendet werden.

In einer speziellen Ausführungsform der Erfindung wird die erfindungsgemäße medizinische Auflage zur Behandlung von Patienten mit (Haut)transplantaten und hierbei insbesondere zur Behandlung von schlecht perfundierten Lappenplastiken eingesetzt. Die beiden vorab genannten hämodynamischen Größen, die arterielle Durchblutung sowie der venöse Rückfluss, stellen auch essenzielle Parameter des Therapieerfolgs chirurgischer Lappenplastiken dar. Als Lappenplastiken werden operative plastisch-chirurgische Techniken bezeichnet, die Haut und/oder Gewebe von einer (entbehrlichen) Stelle des gleichen Individuums an eine neue gewünschte Stelle bringen. In der Regel handelt es sich um reine Hautlappen, es kann aber jedes Gewebe mit oder ohne Haut sowohl gestielt (also mit seinen zugehörigen blutversorgenden Gefäßen und Nerven) als auch frei (d. h. mit Anschluss der Blutgefäße an die Blutversorgung der neuen Umgebung) verpflanzt werden. Die funktionelle Akzeptanz des verpflanzten Gewebes ist dabei ausschließlich von der arteriellen Blutversorgung sowie einem geregelten venösem Abfluss abhängig. Eine NO-bedingte arterielle Vasodilatation verbessert die Durchblutung und somit die notwendige Versorgung der Lappenplastik und durch die antithrombogene Wirkung des NO wird ein venöser Abfluss bzw. Rückfluss des Blutes gefördert und erleichtert. Von außen eingesetzte NO-Präparate können daher den Erfolg einer auf Lappenplastik basierten Therapieoption sichern bzw. fördern.

In einem besonderen Aspekt stellt die Erfindung die folgenden Ausführungsformen bereit:
Ausführungsform 1: Eine aus mindestens zwei Modulen aufgebaute Vorrichtung beliebiger Dimension und Fläche, die mittels der Emission elektromagnetischer Strahlung aus einem der Module (im folgenden als SEM = Strahlungemittierendes Modul bezeichnet) die in einem vorzugsweise dazu enganliegenden zweiten Modul (im folgenden als NOM = NO-Modul bezeichnet) enthaltenen photo- oder redox--labilen Stickstoffmonoxidvorstufen (im Folgenden als NOD = NO-Derivate bezeichnet) vorzugsweise photolytisch spalten kann, sodass im NOM Stickstoffmonoxid (NO) generiert wird und vorzugsweise aus dem NOM freigesetzt werden kann.
Ausführungsform 2: Vorrichtung nach Ausführungsform 1, dadurch gekennzeichnet, dass das NOM vorzugsweise ein integraler, fest mit dem SEM verbundener Bestandteil der Gesamtvorrichtung ist, beinhaltend die Möglichkeit, dass das SEM und das NOM aber auch räumlich von einander getrennt sein können, so dass die Bestrahlung des NOM nicht in direktem aufgelegten Kontakt zum SEM erfolgt, sondern mit einem räumlichen Abstand zu diesem.
Ausführungsform 3: Vorrichtung nach Ausführungsform 1 und 2, dadurch gekennzeichnet, dass für eine optimale Spaltung der in dem NOM eingelagerten photolabilen NO-Vorstufen das SEM elektromagnetische Strahlung mit Wellenlängen emittieren kann, die allein oder mit Unterstützung chemischer, physikalischer oder biologischer Verfahren eine Spaltung von Stickstoffmonoxid-Vorstufen und dadurch eine Freisetzung von Stickstoffmonoxid induzieren kann, wobei durch die Anwesenheit von weiteren Substanzen mit katalytischen Eigenschaften oder spezifischen lichtakzeptorischen Eigenschaften die NO Generierung erleichtert oder gar erst ermöglicht werden kann (z. B. mit Hilfe von Übergangsmetallen wie z. B. Cu2+-lonen, Chromophoren und anderen Substanzen, mit deren Hilfe z. B. auch elektromagnetische Strahlung außerhalb des UVA-Spektrumsbereiches in der Lage sein kann, aus den entsprechenden NO-bildenden Agenzien eine NO-Freisetzung zu ermöglichen).
Ausführungsform 4: Vorrichtung nach Ausführungsformen 1 bis 3, dadurch gekennzeichnet, dass die in dem SEM integrierte Quelle der elektromagnetischen Strahlung eine mit entsprechenden Fluorochromen beschichtete Glüh- oder Entladungslampe (niedrigdruck- oder hochdruckentladend), Licht-emittierende Diode (LED), organische Licht-emittierende Diode (OLED) oder LASER sein kann oder auch jede andere elektromagnetische Strahlungsquelle, die in der Lage ist, aus entsprechenden chemischen Vorstufen bzw. Substraten NO zu generieren.
Ausführungsform 5: Vorrichtung nach Ausführungsformen 1 bis 4, dadurch gekennzeichnet, dass die Quelle der elektromagnetischen Strahlung der SEM mit einer elektronischen Steuerungseinheit der Lichtquelle räumlich kompakt in einem Teil/Gehäuse verbaut sein kann oder von dieser Steuerungseinheit räumlich getrennt sein kann und lediglich über eine Drahtverbindung verbunden sein oder gar gänzlich getrennt sein kann, wobei in diesem Fall die Steuerung der Lichtquelle durch die Steuerungseinheit ferngesteuert stattfinden kann.
Ausführungsform 6: Vorrichtung nach Ausführungsformen 1 bis 5, dadurch gekennzeichnet, dass die NO-Erzeugung im bzw. aus dem NOM einem elektronisch geregelten Sicherheitsmanagement unterliegt, welches 1.) vorzugsweise anhand der Manipulation verschiedener technischer, physikalischer oder chemischer Einstellparameter an dem SEM oder auch NOM geregelt wird, 2.) über sicherheits- und behandlungsrelevante Sensorik (z. B. für NO, NO₂, Temperatur, Lichtstärke, Hautrötung, Zeitabschaltung, usw.) verfügt, 3.) über Anbindungs- und Verbindungsmöglichkeiten zu externen Geräten (z. B. Computer, Smartphones, usw.) verfügt sowie über Software-gesteuerte Applikationen ferngesteuert und angesteuert werden könnte, wobei ein wesentlicher Teil des Sicherheitsmanagements der Vorrichtung eine anwendungs- und benutzerspezifische und elektronisch gesteuerte Erkennung und Benutzungsakzeptanz des spezifisch mit NODs beladenen NOM bzw. deren auswechselbaren bzw. austauschbaren und die photolabile NO-Derivate enthaltenden Teile ist.
Ausführungsform 7: dadurch gekennzeichnet, dass sie zur Anregung des Stoffwechsels von Geweben durch äußerliche Anwendung genutzt werden kann, im Bereich der Dermatologie und Chirurgie zur Behandlung von chirurgischen oder unfallbedingten Wunden, chronischen, nicht- bzw. schlechtheilenden und/oder bakteriell und/oder durch Pilze befallenen Wunden dienen kann, zur Behandlung von dermatologischen Erkrankungen aus dem Formenkreis der entzündlichen, immunologisch gesteuerten bzw. Autoimmunerkrankungen verwendet werden kann, so z. B. zur Behandlung diabetischer Füße und Wunden, neuropathischer Schmerzen beim Diabetes und anderen Erkrankungen, von Krampfadern, lokaler oberflächlicher als auch tiefsitzender Ischämien und thrombopathischer Erkrankungen von Geweben, akuter und chronischer Entzündungen der Haut, von Allergien der Haut, parasitärer Infektionen der Haut, der atopischen Dermatitis insbesondere Neurodermitis, Dermatomyositis, Pemphigus vulgaris und/oder anderer lokaler und systemischer Infektionen und/oder akuter und chronischer Entzündungssituationen, von Wunddefekten, wie dem chronischen diabetischneuropathischen Ulcus, Ulcus cruris, Dekubituswunden, sekundär heilenden infizierten Wunden, reizlosen, primär heilende Wunden, wie insbesondere ablative Riss- oder Schürfwunden, (Haut-)Transplantaten aber auch zur Behandlung größerer Körperareale zur Therapie systemischer Erkrankungen wie z. B. des erhöhten Blutdrucks (Hypertonie) und verwandten hämodynamischen Erkrankungen, wobei eine Behandlung individuell zwischen wenigen Sekunden und vielen Stunden dauern kann, wobei zum Zweck einer Behandlung das NOM auf das zu exponierende Areal aufgelegt wird und vorzugsweise im direkten Kontakt oder aber auch aus einer räumliche Entfernung der aus der SEM emittierten elektromagnetischen Strahlung ausgesetzt wird, wobei eine solche Behandlung zwischen wenigen Sekunden und vielen Stunden dauern kann.

### BEZUGSZEICHEN

- 1: innere Schicht
- 2: mittlere Schicht
- 3: äußere Schicht
- 4: Rückschicht
- 5: transparentes Fenster in der Rückschicht
- 6: Befestigungsvorrichtung für SEM
- 7: nicht-adhäsiver Teil einer inneren Schicht
- 8: Hohlraum im inneren Bereich der Auflage
- 9: Strahlungsquelle
- 10, 10': Abstandshalter

### FIGUREN

Die Erfindung wird nachfolgend anhand der Figuren näher erläutert ohne die Erfindung auf dieses zu beschränken. Es zeigen:

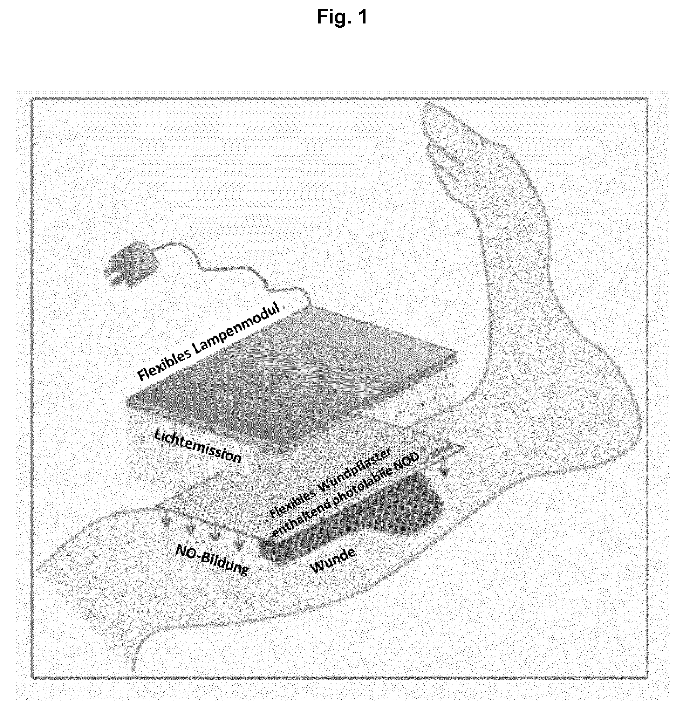

- Fig. 1:: die Anwendung zur Wundbehandlung, bei der das NOM als flexible Auflage auf die Beinwunde aufgelegt wird und über ein darüber positioniertes SEM mit UV-Strahlung bestrahlt wird, so dass das NOM körperseitig NO freisetzt.

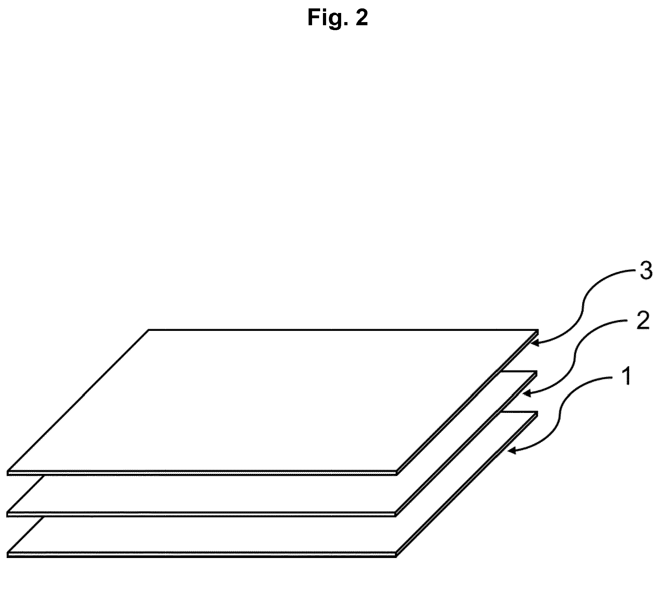

- Fig. 2:: eine schematische Explosionszeichnung einer Ausführungsform der NOM der medizinischen Auflage mit innerer Schicht (1), mittlerer Schicht (2) und äußerer Schicht (3).

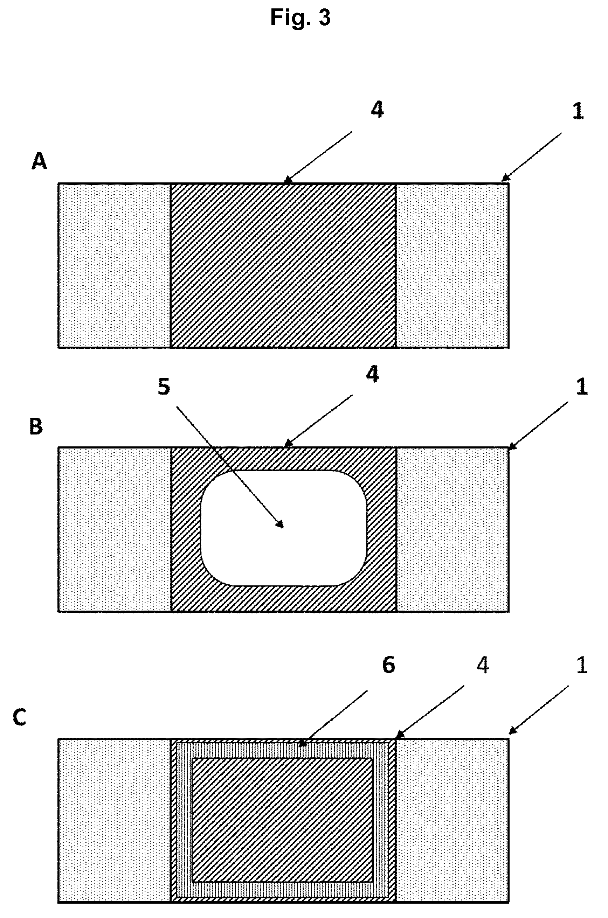

- Fig. 3:: eine schematische Darstellung eines mehrschichtigen NOM in Aufsicht mit der inneren Schicht (1) und der Rückschicht (4) in **(A),** mit einem zusätzlichen transparenten Bereich als Aktivierungsfenster in **(B)** und einer Befestigungsvorrichtung (z.B. als Klettverschluss) für das SEM in **(C).**

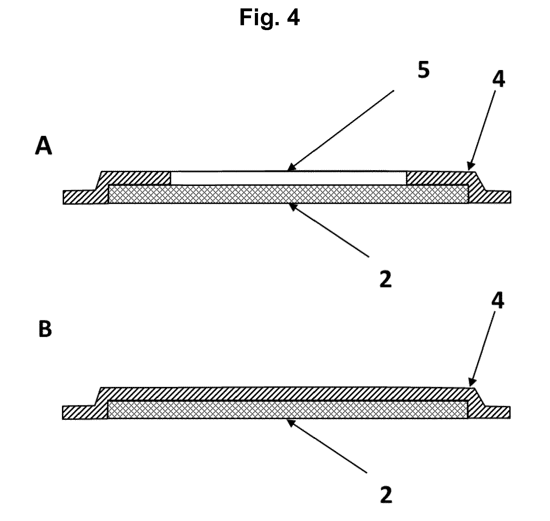

- Fig. 4:: eine schematische Darstellung eines zweischichtigen NOM im Querschnitt Aufsicht mit der mittleren Schicht (2) und der Rückschicht (4) in **(B),** mit einem zusätzlichen transparenten Bereich als Aktivierungsfenster in **(A).**

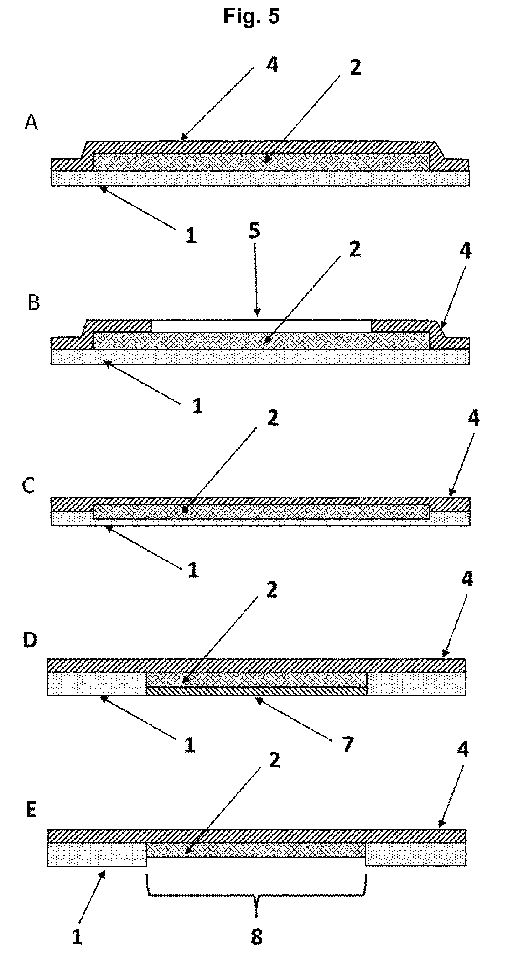

- Fig. 5:: eine schematische Darstellung eines dreischichtigen NOM im Querschnitt Aufsicht mit der inneren Schicht (1), der mittleren Schicht (2) und der Rückschicht (4) in **(A),** mit einem zusätzlichen transparenten Bereich als Aktivierungsfenster in **(B),** mit einer flächig eingelagerten inneren Schicht in **(C),** mit einer im Bereich der mittleren Schicht nicht-adhäsiven inneren Schicht in **(D),** und mit einer allseitig umschlossenen Hohlraum unterhalb der mittleren Schicht in **(E).**

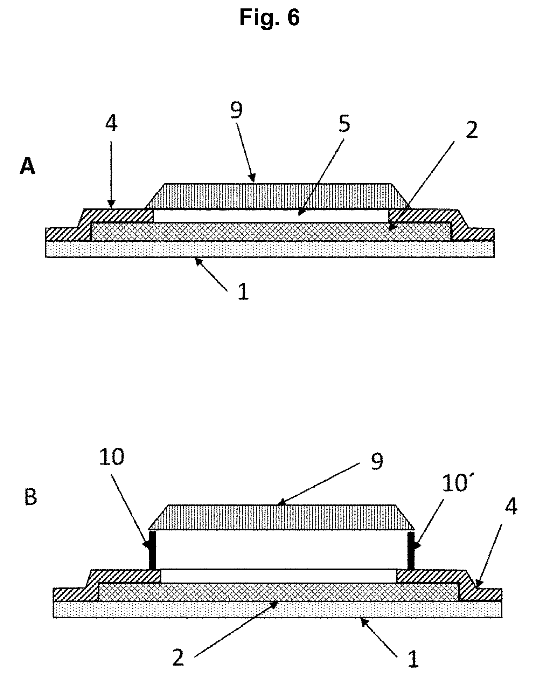

- Fig. 6:: eine schematische Darstellung der medizinischen Auflage im Querschnitt mit einem dreischichtigen NOM t mit der inneren Schicht (1), der mittleren Schicht (2) und der Rückschicht (4) mit transparentem Aktivierungsfenster (5) und der aufgelagerten Strahlungsquelle als SOM in **(A),** mit zusätzlichen Abstandshaltern (10, 10') in **(B).**

### LITERATUR

[1] K.D. Kröncke, K. Fehsel, and V. Kolb-Bachofen, Inducible nitric oxide synthase in human diseases. Clin Exp Immunol 113 (1998) 147-56.
[2] K. Matsunaga, and R.F. Furchgott, Responses of rabbit aorta to nitric oxide and superoxide generated by ultraviolet irradiation of solutions containing inorganic nitrite. J Pharmacol Exp Ther 259 (1991) 1140-6.
[3] M. Fischer, and P. Warneck, Photodecomposition of nitrite and undissociated nitrous acid in aqueous solution. J.Phys.Chem. 100 (1996) 18749-18756.
[4] H. Strehlow, and I. Wagner, Flash photolysis in aqueous nitrite solutions. Z. Phys. Chem. NF 132 (1982) 151-160.
[5] S. Frank, H. Kampfer, C. Wetzler, and J. Pfeilschifter, Nitric oxide drives skin repair: novel functions of an established mediator. Kidney Int 61 (2002) 882-8.
[6] S. Frank, B. Stallmeyer, H. Kampfer, N. Kolb, and J. Pfeilschifter, Nitric oxide triggers enhanced induction of vascular endothelial growth factor expression in cultured keratinocytes (HaCaT) and during cutaneous wound repair. Faseb J 13 (1999) 2002-14.
[7] S. Frank, H. Kampfer, M. Podda, R. Kaufmann, and J. Pfeilschifter, Identification of copper/zinc superoxide dismutase as a nitric oxide- regulated gene in human (HaCaT) keratinocytes: implications for keratinocyte proliferation. Biochem J 346 Pt 3 (2000) 719-28.
[8] K. Yamasaki, H.D. Edington, C. McClosky, E. Tzeng, A. Lizonova, I. Kovesdi, D.L. Steed, and T.R. Billiar, Reversal of impaired wound repair in iNOS-deficient mice by topical adenoviral-mediated iNOS gene transfer. J Clin Invest 101 (1998) 967-71.
[9] J. Pfeilschifter, W. Eberhardt, and A. Huwiler, Nitric oxide and mechanisms of redox signalling: matrix and matrix-metabolizing enzymes as prime nitric oxide targets. Eur J Pharmacol 429 (2001) 279-86.
[10] Y. Ishii, T. Ogura, M. Tatemichi, H. Fujisawa, F. Otsuka, and H. Esumi, Induction of matrix metalloproteinase gene transcription by nitric oxide and mechanisms of MMP-1 gene induction in human melanoma cell lines. Int J Cancer 103 (2003) 161-8.
[11] M.B. Witte, F.J. Thornton, D.T. Efron, and A. Barbul, Enhancement of fibroblast collagen synthesis by nitric oxide. Nitric Oxide 4 (2000) 572-82.
[12] F. Verrecchia, and A. Mauviel, TGF-beta and TNF-alpha: antagonistic cytokines controlling type I collagen gene expression. Cell Signal 16 (2004) 873-80.
[13] D.A. Siwik, and W.S. Colucci, Regulation of matrix metalloproteinases by cytokines and reactive oxygen/nitrogen species in the myocardium. Heart Fail Rev 9 (2004) 43-51.
[14] V.M. Darley-Usmar, R.P. Patel, V.B. O'Donnell, and B.A. Freeman, Antioxidant actions of nitric oxide. in: L.J. Ignarro, (Ed.), Nitric Oxide: Biology and Pathobiology, Academic Press, San Diego, 2000, pp. 265-276.
[15] S.P. Goss, B. Kalyanaraman, and N. Hogg, Antioxidant effects of nitric oxide and nitric oxide donor compounds on low-density lipoprotein oxidation. Methods Enzymol 301 (1999) 444-53.
[16] D.A. Wink, J.A. Cook, R. Pacelli, J. Liebmann, M.C. Krishna, and J.B. Mitchell, Nitric oxide (NO) protects against cellular damage by reactive oxygen species. Toxicol Lett 82-83 (1995) 221-6.
[17] B. Brüne, A. von Knethen, and K.B. Sandau, Nitric oxide (NO): an effector of apoptosis. Cell Death Differ 6 (1999) 969-75.
[18] D. Moellering, J. McAndrew, R.P. Patel, T. Cornwell, T. Lincoln, X. Cao, J.L. Messina, H.J. Forman, H. Jo, and V.M. Darley-Usmar, Nitric oxide-dependent induction of glutathione synthesis through increased expression of gamma-glutamylcysteine synthetase. Arch Biochem Biophys 358 (1998) 74-82.
[19] U. Forstermann, M. Nakane, W.R. Tracey, and J.S. Pollock, Isoforms of nitric oxide synthase: functions in the cardiovascular system. Eur Heart J 14 Suppl I (1993) 10-5.
[20] P. He, M. Zeng, and F.E. Curry, Effect of nitric oxide synthase inhibitors on basal microvessel permeability and endothelial cell [Ca2+]i. Am J Physiol 273 (1997) H747-55.
[21] M. Toborek, and S. Kaiser, Endothelial cell functions. Relationship to atherogenesis. Basic Res Cardiol 94 (1999) 295-314.
[22] M. Keim, and B. Strauer, Endotheliale Dysfunktion; Therapeutische und prognostische Relevanz. Internist. 40 (1999) 1300-1307.
[23] T.P. Amadeu, A.B. Seabra, M.G. de Oliveira, and A.M. Costa, S-nitrosoglutathione-containing hydrogel accelerates rat cutaneous wound repair. J Eur Acad Dermatol Venereol 21 (2007) 629-37.
[24] R. Weller, and M.J. Finnen, The effects of topical treatment with acidified nitrite on wound healing in normal and diabetic mice. Nitric Oxide 15 (2006) 395-9.
[25] A.B. Shekhter, V.A. Serezhenkov, T.G. Rudenko, A.V. Pekshev, and A.F. Vanin, Beneficial effect of gaseous nitric oxide on the healing of skin wounds. Nitric Oxide 12 (2005) 210-9.
[26] W.S. McDonald, T.P. Lo, Jr., M. Thurmond, C. Jones, R. Cohen, A. Miller, and D. Beasley, Role of nitric oxide in skin flap delay. Plast Reconstr Surg 113 (2004) 927-31.
[27] C. Beige, P.B. Massion, M. Pelat, and J.L. Balligand, Nitric oxide and the heart: update on new paradigms. Ann N Y Acad Sei 1047 (2005) 173-82.
[28] B. Gaston, Summary: systemic effects of inhaled nitric oxide. Proc Am Thorac Soc 3 (2006) 170-2.
[29] T.M. Dawson, and S.H. Snyder, Gases as biological messengers: nitric oxide and carbon monoxide in the brain. J Neurosci 14 (1994) 5147-59.
[30] C.C. Miller, M.K. Miller, A. Ghaffari, and B. Kunimoto, Treatment of chronic nonhealing leg ulceration with gaseous nitric oxide: a case study. J Cutan Med Surg 8 (2004) 233-8.
[31] A. Ghaffari, D.H. Neil, A. Ardakani, J. Road, A. Ghahary, and C.C. Miller, A direct nitric oxide gas delivery system for bacterial and mammalian cell cultures. Nitric Oxide 12 (2005) 129-40.
[32] A. Ghaffari, C.C. Miller, B. McMullin, and A. Ghahary, Potential application of gaseous nitric oxide as a topical antimicrobial agent. Nitric Oxide 14 (2006) 21-9.
[33] A. Ghaffari, R. Jalili, M. Ghaffari, C. Miller, and A. Ghahary, Efficacy of gaseous nitric oxide in the treatment of skin and soft tissue infections. Wound Repair Regen 15 (2007) 368-77.
[34] Z.S. Galis, and J.J. Khatri, Matrix metalloproteinases in vascular remodeling and atherogenesis: the good, the bad, and the ugly. Circ Res 90 (2002) 251-62.
[35] S.C. Tyagi, and M.R. Hayden, Role of nitric oxide in matrix remodeling in diabetes and heart failure. Heart Fail Rev 8 (2003) 23-8.
[36] J. Pfeilschifter, W. Eberhardt, and K.F. Beck, Regulation of gene expression by nitric oxide. Pflugers Arch 442 (2001) 479-86.
[37] R. Zamora, Y. Vodovotz, K.S. Aulak, P.K. Kim, J.M. Kane, 3rd, L. Alarcon, D.J. Stuehr, and T.R. Billiar, A DNA microarray study of nitric oxide-induced genes in mouse hepatocytes: implications for hepatic heme oxygenase-1 expression in ischemia/reperfusion. Nitric Oxide 7 (2002) 165-86.
[38] J. Hemish, N. Nakaya, V. Mittal, and G. Enikolopov, Nitric oxide activates diverse signaling pathways to regulate gene expression. J Biol Chem 278 (2003) 42321-9.
[39] M. Ziche, L. Morbidelli, E. Masini, S. Amerini, H.J. Granger, C.A. Maggi, P. Geppetti, and F. Ledda, Nitric oxide mediates angiogenesis in vivo and endothelial cell growth and migration in vitro promoted by substance P. J Clin Invest 94 (1994) 2036-44.
[40] S.J. Leibovich, P.J. Polverini, T.W. Fong, L.A. Harlow, and A.E. Koch, Production of angiogenic activity by human monocytes requires an L-arginine/nitric oxide-synthasedependent effector mechanism. Proc Natl Acad Sci U S A 91 (1994) 4190-4.
[41] N.S. Bryan, B.O. Fernandez, S.M. Bauer, M.F. Garcia-Saura, A.B. Milsom, T. Rassaf, R.E. Maloney, A. Bharti, J. Rodriguez, and M. Feelisch, Nitrite is a signaling molecule and regulator of gene expression in mammalian tissues. Nature Chemical Biology 1 (2005) 290-297.

## Patentansprüche

1. Medizinische Auflage umfassend
a. ein Strahlung-emittierendes Modul (SEM) mit einer Strahlungsquelle zur Emission elektromagnetischer Strahlung, und
b. ein NO-Modul (NOM), das photolabile Stickstoffmonoxidvorstufen (NOD) enthält, wobei die aus dem SEM emittierte elektromagnetische Strahlung diese NOD spalten kann, so dass im NOM NO generiert wird, welches aus dem NOM freigesetzt werden kann;
wobei in der Auflage ein System eingesetzt wird, welches mehrfach oxidierte Stickoxide, Sauerstoffradikalanionen, aquatisierte Elektronen oder Hydroxylradikale abbaut oder neutralisiert (Radikalfänger-System).

2. Medizinische Auflage gemäß Anspruch 1, **dadurch gekennzeichnet, dass** sie zusätzlich Übergangsmetallkationen enthält, die bevorzugt Cu²⁺ Ionen sind.

3. Medizinische Auflage gemäß Anspruch 2, **dadurch gekennzeichnet, dass** die elektromagnetische Strahlung eine Wellenlänge zwischen 400 bis 470 nm und bevorzugt zwischen 400 und 450 nm aufweist.

4. Medizinische Auflage gemäß Anspruch 1 bis 3, **dadurch gekennzeichnet, dass** das System, welches mehrfach oxidierte Stickoxide, Sauerstoffradikalanionen, aquatisierte Elektronen oder Hydroxylradikale abbaut oder neutralisiert, ausgewählt ist aus der Gruppe bestehend aus Ascorbinsäure, Ascorbat, Vitamin E, Derivate von Vitamin E, Thiole, Radikalfänger, Sauerstoffspezies oder Stickstoffspezies abbauende Enzyme.

5. Medizinische Auflage gemäß einem der vorherigen Ansprüche, **dadurch gekennzeichnet, dass** sie eine medizinische Wundauflage ist.

6. Medizinische Auflage gemäß einem der vorherigen Ansprüche, **dadurch gekennzeichnet, dass** das NOM fest mit SEM verbunden ist.

7. Medizinische Auflage gemäß einem der Ansprüche 1 bis 5, **dadurch gekennzeichnet, dass** das NOM in einem räumlichen Abstand zur SEM angeordnet ist.

8. Medizinische Auflage gemäß einem der vorherigen Ansprüche, **dadurch gekennzeichnet, dass** das NOM als leicht austauschbarer Verbrauchsartikel ausgestaltet ist.

9. Medizinische Auflage gemäß einem der vorherigen Ansprüche, **dadurch gekennzeichnet, dass** die elektromagnetische Strahlungsquelle der SEM mit einer elektronischen Steuerungseinheit dieser Quelle, bevorzugt räumlich kompakt, in einem Gehäuse verbaut ist.

10. Medizinische Auflage gemäß einem der vorherigen Ansprüche, **dadurch gekennzeichnet, dass** die elektromagnetische Strahlungsquelle der SEM von der elektronischen Steuerungseinheit dieser Quelle räumlich getrennt ist und die Steuerung über eine Drahtverbindung oder über eine Fernsteuerung erfolgt.

11. Medizinische Auflage gemäß einem der vorherigen Ansprüche, **dadurch gekennzeichnet, dass** die elektromagnetische Strahlungsquelle ausgewählt ist aus der Gruppe enthaltend eine mit entsprechenden Fluorochromen beschichtete Glüh- oder Gasentladungslampe (niedrigdruck- oder hochdruckentladend), eine Licht-emittierende Diode (LED), eine organische Licht-emittierende Diode (OLED), und ein Laser.

12. Medizinische Auflage gemäß einem der vorherigen Ansprüche, **dadurch gekennzeichnet, dass** die photolabilen NOD ausgewählt sind aus der Gruppe enthaltend organische Nitrate, anorganische Nitrate, Nitrite, Schwefel-, Stickstoff- oder Sauerstoff-Nitrosoverbindungen, NO-Metall-Verbindungen und NO-chelatierende Substanzen.

13. Medizinische Auflage gemäß einem der vorherigen Ansprüche, **dadurch gekennzeichnet, dass** die photolabilen NOD ein anorganisches Nitrit sind.

14. Medizinische Auflage gemäß einem der vorherigen Ansprüche, **dadurch gekennzeichnet, dass** die photolabilen NOD ausgewählt sind aus der Gruppe bestehend aus LiNO₂, NaNO₂, KNO₂, RbNO₂, CsNO₂, FrNO₂, Be(NO₂)₂, Mg(NO₂)₂, Ca(NO₂)₂ Sr(NO₂)₂, Ba(NO₂)₂ oder Ra(NO₂)₂.

15. Medizinische Auflage gemäß einem der vorherigen Ansprüche, **dadurch gekennzeichnet, dass** sie NaNO₂ als photolabile NO-Vorstufe enthält und Ascorbinsäure oder Ascorbat als Radikalfänger-System.

16. Medizinische Auflage gemäß einem der vorherigen Ansprüche, **dadurch gekennzeichnet, dass** sie über eine sicherheits- und behandlungsrelevante Sensorik verfügt, bevorzugt für NO, NO₂, Temperatur, Lichtstärke, Hautrötung, oder Zeitabschaltung.

17. Medizinische Auflage gemäß einem der vorherigen Ansprüche zur Verwendung bei der Behandlung von Erkrankungen mittels Auflegen oder Aufkleben auf das zu exponierende Areal.

18. Medizinische Auflage gemäß Anspruch 17, **dadurch gekennzeichnet, dass** die Behandlung ausgewählt ist aus der Gruppe enthaltend:
a.Behandlung diabetischer Füße und Wunden;
b.Behandlung neuropathischer Schmerzen;
c. Behandlung von Krampfadern;
d.Behandlung von lokalen oberflächlichen oder tiefsitzenden Ischämien und thrombopathischer Erkrankungen von Geweben;
e.Behandlung akuter und chronischer Entzündungen der Haut;
f. Behandlung von Allergien der Haut;
g.Behandlung von parasitären Infektionen der Haut;
h.Behandlung der atopischen Dermatitis insbesondere Neurodermitis, Dermatomyositis und Pemphigus vulgaris;
i. Behandlung von Wunddefekten, wie dem chronischen diabetischneuropathischen Ulcus, Ulcus cruris, Dekubituswunden;
j. Behandlung sekundär heilender infizierter Wunden;
k. Behandlung primär heilender Wunden, wie ablative Riss- oder Schürfwunden;
l. Behandlung größerer Körperareale zur Therapie systemischer Erkrankungen wie z. B. des erhöhten Blutdrucks (Hypertonie) und verwandten hämodynamischen Erkrankungen;
m. Behandlung von Patienten mit (Haut)transplantaten;
n.Behandlung des diabetischen Schmerzes der unteren Extremitäten (Fuß oder Bein); und
o.Behandlung von schlecht perfundierten Lappenplastiken.

19. Medizinische Auflage gemäß Anspruch 17, **dadurch gekennzeichnet, dass** sie zur Behandlung chronischer Wunden der unteren Extremitäten von Diabetikern verwendet wird.

20. Medizinische Auflage gemäß Anspruch 17 bis 19, **dadurch gekennzeichnet, dass** die Behandlung zwischen wenigen Sekunden und vielen Stunden dauern kann.

21. Medizinische Auflage gemäß Anspruch 20, **dadurch gekennzeichnet, dass** die Behandlung zwischen 5 und 30 Minuten, bevorzugt zwischen 7,5 bis 20 Minuten und besonders bevorzugt zwischen 10 bis 15 Minuten dauert.

## Claims

1. A medical dressing comprising
a. a radiation-emitting module having a source of radiation for the emission of electromagnetic radiation, and
b. an NO module (NOM) containing photolabile nitric oxide donors (NOD), whereby the electromagnetic radiation stemming from the radiation-emitting module can cleave these nitric oxide donors, so that the NO module generates NO that can then be released from the NO module;
whereby in the dressing, a system is used that degrades or neutralizes polyoxidized nitrogen oxides, oxygen radical anions, hydrated electrons or hydroxyl radicals (radical trapping system).

2. The medical dressing according to claim 1, **characterized in that** it additionally contains transition metal cations, which are preferably Cu²⁺ ions.

3. The medical dressing according to claim 2, **characterized in that** the electromagnetic radiation has a wavelength ranging from 400 nm to 470 nm and preferably between 400 nm and 450 nm.

4. The medical dressing according to claims 1 to 3, **characterized in that** the system that breaks down or neutralizes polyoxidized nitrogen oxides, oxygen radical anions, hydrated electrons or hydroxyl radicals is selected from the group consisting of ascorbic acid, ascorbate, vitamin E, derivatives of vitamin E, thiols, radical traps, and enzymes that break down oxygen species or nitrogen species.

5. The medical dressing according to one of the preceding claims, **characterized in that** it is a medical wound dressing.

6. The medical dressing according to one of the preceding claims, **characterized in that** the NO module is permanently joined to the radiation-emitting module.

7. The medical dressing according to one of claims 1 to 5, **characterized in that** the NO module is arranged at a certain distance from the radiation-emitting module.

8. The medical dressing according to one of the preceding claims, **characterized in that** the NO module is configured as an easily replaced disposable article.

9. The medical dressing according to one of the preceding claims, **characterized in that,** along with an electronic control unit for the light source, the source of electromagnetic radiation of the radiation-emitting module is installed in a housing, preferably in a compact manner.

10. The medical dressing according to one of the preceding claims, **characterized in that** the source of electromagnetic radiation of the radiation-emitting module is physically separated from the electronic control unit for this source, and the control is carried out via a wired connection or by remote control.

11. The medical dressing according to one of the preceding claims, **characterized in that** the source of electromagnetic radiation is selected from the group comprising a glow-discharge or gas-discharge lamp (low-pressure discharging or high-pressure discharging) coated with appropriate fluorochromes, a light-emitting diode (LED), an organic light-emitting diode (OLED) and a laser.

12. The medical dressing according to one of the preceding claims, **characterized in that** the photolabile nitric oxide donors (NOD) are selected from the group containing organic nitrates, inorganic nitrates, nitrites, sulfur-nitroso, nitrogen-nitroso or oxygen-nitroso compounds, NO-metal compounds and NO-chelating substances.

13. The medical dressing according to one of the preceding claims, **characterized in that** the photolabile nitric oxide donor (NOD) is an inorganic nitrite.

14. The medical dressing according to one of the preceding claims, **characterized in that** the photolabile nitric oxide donors (NOD) are selected from the group consisting of LiNO₂, NaNO₂, KNO₂, RbNO₂, CsNO₂, FrNO₂, Be(NO₂)₂, Mg(NO₂)₂, Ca(NO₂)₂, Sr(NO₂)₂, Ba(NO₂)₂ or Ra(NO₂)₂.

15. The medical dressing according to one of the preceding claims, **characterized in that** it contains NaNO₂ as the photolabile nitric oxide donor and ascorbic acid or ascorbate as the radical trapping system.

16. The medical dressing according to one of the preceding claims, **characterized in that** it has a safety-relevant and treatment-relevant sensor system, preferably for NO, NO₂, temperature, light intensity, skin reddening, or time switch-OFF.

17. The medical dressing according to one of the preceding claims for use in the treatment of diseases by placing or adhering the medical dressing onto the region that is to be exposed.

18. The medical dressing according to claim 17, **characterized in that** the treatment is selected from the group comprising:
a. treatment of diabetic feet and wounds;
b. treatment of neuropathic pain;
c. treatment of varicose veins;
d. treatment of local superficial as well as deep ischemias and thrombopathic diseases of the tissues;
e. treatment of acute and chronic inflammation of the skin;
f. treatment of skin allergies;
g. treatment of parasitic infections of the skin;
h. treatment of atopic dermatitis, especially neurodermititis, dermatomyositis and *Pemphigus vulgaris;*
i. treatment of wound defects, such as chronic diabetic-neuropathic *Ulcus, Ulcus cruris*, decubitus wounds;
j. treatment of infected wounds healing by second intention;
k. treatment of wounds healing by first intention, such as ablative lacerations or abrasions;
l. treatment of larger areas of the body for the therapy of systemic diseases such as, for example, high blood pressure (hypertonia) and related hemodynamic diseases;
m. treatment of patients with (skin) transplants;
n. treatment of diabetic pain in the lower extremities (foot or leg); and
o. treatment in cases of poorly perfused skin flap plastic surgeries.

19. The medical dressing according to claim 17, **characterized in that** it is used for the treatment of chronic wounds in the lower extremities of diabetic patients.

20. The medical dressing according to claims 17 to 19, **characterized in that** the treatment can last between a few seconds and many hours.

21. The medical dressing according to claim 20, **characterized in that** the treatment lasts between 5 and 30 minutes, preferably between 7.5 and 20 minutes and especially preferably between 10 and 15 minutes.

## Revendications

1. Emplâtre médical, comprenant :
a. un module émetteur de rayonnement (SEM) avec une source émettrice de rayonnement électromagnétique et
b. un module NO (NOM) qui contient des précurseurs photolabiles de monoxyde d'azote (NOD), le rayonnement électromagnétique émis par le SEM ayant la capacité scinder ces NOD, de sorte que du NO soit généré dans le NOM et que du NO puisse être libéré du NOM,
dans l'emplâtre étant mis en place un système qui dégrade ou neutralise des polyoxydes d'azote, des anions radicaux d'oxygène, des électrons hydratés ou des radicaux hydroxyles (système de capteurs de radicaux).

2. Emplâtre médical selon la revendication 1, **caractérisé en ce qu'**il contient également des cations métalliques de transition qui sont de préférence des ions Cu²⁺.

3. Emplâtre médical selon la revendication 2, **caractérisé en ce que** le rayonnement électromagnétique présente une longueur d'onde comprise entre 400 et 470 nm et préférentiellement entre 400 et 450 nm.

4. Emplâtre médical selon la revendication 1 à 3, **caractérisé en ce que** le système qui dégrade ou neutralise des poly-oxydes d'azote, des anions radicaux d'oxygène, des électrons hydratés ou des radicaux hydroxyles est choisi dans le groupe composé d'acide ascorbique, d'ascorbate, de vitamine E, de dérivés de vitamine E, de thiols, de capteurs de radicaux, d'enzymes dégradant les espèces d'oxygène ou les espèces d'azote.

5. Emplâtre médical selon l'une des revendications précédentes, **caractérisé en ce que** qu'il constitue un emplâtre médical pour blessures.

6. Emplâtre médical selon l'une des revendications précédentes, **caractérisé en ce que** le NOM est solidaire du SEM.

7. Emplâtre médical selon l'une des revendications 1 à 5, **caractérisé en ce que** le NOM est spatialement distant du SEM.

8. Emplâtre médical selon l'une des revendications précédentes, **caractérisé en ce que** le NOM est conçu comme consommable facilement remplaçable.

9. Emplâtre médical selon l'une des revendications précédentes, **caractérisé en ce que** la source de rayonnement électromagnétique des SEM est montée dans un boîtier avec une unité de commande électronique, de préférence sous forme compacte, de cette source..

10. Emplâtre médical selon l'une des revendications précédentes, **caractérisé en ce que** la source de rayonnement électromagnétique des SEM est séparée spatialement de l'unité de commande électronique de cette source et **en ce que** la commande a lieu via une liaison filaire ou via une télécommande.

11. Emplâtre médical selon l'une des revendications précédentes, **caractérisé en ce que** la source de rayonnement électromagnétique est choisie dans le groupe comprenant une lampe à incandescence ou à décharge de gaz revêtue de fluorochromes appropriés (décharge à basse pression ou décharge à haute pression), une diode électroluminescente (LED), une diode organique électroluminescente (OLED) et un laser.

12. Emplâtre médical selon l'une des revendications précédentes, **caractérisé en ce que** les NOD photolabiles sont choisis dans le groupe comprenant des nitrates organiques, des nitrates inorganiques, des nitrites, des nitroso-composés de soufre, d'azote ou d'oxygène, des composés de métal et de NO et des substances chélatant le NO.

13. Emplâtre médical selon l'une des revendications précédentes, **caractérisé en ce que** les NOD photolabiles sont un nitrite inorganique.

14. Emplâtre médical selon l'une des revendications précédentes, **caractérisé en ce que** les NOD photolabiles sont choisis dans le groupe composé de LiNO₂, NaNO₂, KNO₂, RbNO₂, CsNO₂, FrNO₂, Be(NO₂)₂, Mg(NO₂)₂, Ca(NO₂)₂, Sr(NO₂)₂, Ba(NO₂)₂, ou Ra(NO₂)₂.

15. Emplâtre médical selon l'une des revendications précédentes, **caractérisé en ce qu'**il contient du NaNO₂ en tant que précurseur de NO photolabile et de l'acide ascorbique ou un ascorbate en tant que système de capteurs de radicaux.

16. Emplâtre médical selon l'une des revendications précédentes, **caractérisé en ce qu'**il dispose de capteurs pertinents pour la sécurité et le traitement, de préférence sensibles à NO, à NO₂, à la température, à la luminosité, à la rougeur cutanée ou au temps.

17. Emplâtre médical selon l'une des revendications précédentes, destiné à être utilisé pour le traitement de maladies par application ou collage sur la zone exposée.

18. Emplâtre médical selon la revendication 17, **caractérisé en ce que** le traitement est choisi dans le groupe comprenant :
a. le traitement de pieds et plaies diabétiques ;
b. le traitement des douleurs neuropathiques ;
c. le traitement des varices ;
d. le traitement des ischémies locales superficielles ou profondes et de maladies thrombotiques tissulaires ;
e. le traitement des inflammations cutanées aiguës et chroniques ;
f. le traitement des allergies cutanées ;
g. le traitement d'infections parasitaires de la peau ;
h. le traitement de la dermatite atopique, et plus particulièrement de la névrodermite, de la dermatomyosite et le pemphigus vulgaire ;
i. le traitement des lésions de plaies, telles que l'ulcère diabétique neuropathique chronique, l'ulcère crural, les escarres;
j. le traitement des blessures infectées du deuxième degré ;
k. le traitement des blessures du premier degré, telles que les blessures ablatives résultant de déchirures ou d'égratignures ;
l. le traitement des zones corporelles plus grandes pour la thérapie de maladies systémiques, telles que, par exemple, la tension artérielle élevée (hypertonie) et les maladies hémodynamiques apparentées ;
m. le traitement des patients avec greffe (cutanée) ;
n. le traitement de la douleur diabétique des extrémités inférieures (pied ou jambe) et
o. le traitement des plasties de lambeau avec mauvaise perfusion.

19. Emplâtre médical selon la revendication 17, **caractérisé en ce qu'**il est utilisé pour le traitement de plaies chroniques des extrémités inférieures des diabétiques.

20. Emplâtre médical selon la revendication 17 à 19, **caractérisé en ce que** le traitement peut durer entre quelques secondes et plusieurs heures.

21. Emplâtre médical selon la revendication 20, **caractérisé en ce que** le traitement dure entre 5 et 30 minutes, préférentiellement entre 7,5 à 20 minutes et, particulièrement entre 10 à 15 minutes.
